# EUROPEAN PATENT SPECIFICATION

(11) **EP 4 135 702 B1**
(45) Date of publication and mention of the grant of the patent: **03.06.2026**
(21) Application number: 21719985.0
(22) Date of filing: 13.04.2021
(51) Int. Cl.: A61K 31/517, A61K 9/10, A61K 9/00, A61K 47/10, A61K 9/08, C07D 247/02, A61P 31/18, A61K 31/519, C07D 401/14, C07D 403/14, C07D 471/04

(54) **INHIBITORS OF HUMAN IMMUNODEFICIENCY VIRUS REPLICATION**
INHIBITOREN DER REPLIKATION DES MENSCHLICHEN IMMUNDEFIZIENZVIRUS
INHIBITEURS DE LA RÉPLICATION DU VIRUS DE L'IMMUNODÉFICIENCE HUMAINE

(30) Priority: 15.04.2020 US 202063010196 P; 22.04.2020 US 202063013576 P
(43) Date of publication of application: 22.02.2023
(73) Proprietor: VIIV Healthcare UK (No.5) Limited, Stevenage SG1 2NY (GB)
(72) Inventor: GILLIS, Eric P., Branford, Connecticut 06405 (US); PARCELLA, Kyle E., Branford, Connecticut 06405 (US); DALWADI, Gautam, Collegeville, Pennsylvania 19426 (US); PARKER, Dawn D., Branford, Connecticut 06405 (US)
(74) Representative: Matley, Joshua Ellis
(86) International application number: PCT/IB2021/053046
(87) International publication number: WO 2021/209900

(56) References cited:
- WO-A1-2020/018459
- WO-A1-2020/157692
- WO-A1-2020/254985

## Description

### FIELD OF THE INVENTION

The invention relates pharmaceutical compositions and their use in the treatment of HIV infection.

### BACKGROUND OF THE INVENTION

Acquired immunodeficiency syndrome (AIDS) is the result of infection by HIV. HIV continues to be a major global public health issue. In 2015, an estimated 36.7 million people were living with HIV (including 1.8 million children) - a global HIV prevalence of 0.8%. The vast majority of this number live in low- and middle- income countries. In the same year, 1.1 million people died of AIDS-related illnesses.

Current therapy for HIV-infected individuals consists of a combination of approved anti-retroviral agents. Close to four dozen drugs are currently approved for HIV infection, either as single agents, fixed dose combinations or single tablet regimens; the latter two containing 2-4 approved agents. These agents belong to a number of different classes, targeting either a viral enzyme or the function of a viral protein during the virus replication cycle. Thus, agents are classified as either nucleotide reverse transcriptase inhibitors (NRTIs), non-nucleotide reverse transcriptase inhibitors (NNRTIs), protease inhibitors (PIs), integrase strand transfer inhibitors (INSTIs), or entry inhibitors (one, maraviroc, targets the host CCR5 protein, while the other, enfuvirtide, is a peptide that targets the gp41 region of the viral gp160 protein). In addition, a pharmacokinetic enhancer (cobicistat or ritonavir) can be used in combinations with antiretroviral agents (ARVs) that require boosting.

Despite the armamentarium of agents and drug combinations, there remains a medical need for new anti-retroviral agents. High viral heterogeneity, drug-associated toxicity, tolerability problems, and poor adherence can all lead to treatment failure and may result in the selection of viruses with mutations that confer resistance to one or more antiretroviral agents or even multiple drugs from an entire class (Beyrer, C., Pozniak A. HIV drug resistance - an emerging threat to epidemic control. N. Engl. J. Med. 2017, 377, 1605-1607; Gupta, R. K., Gregson J., et al. HIV-1 drug resistance before initiation or re-initiation of first-line antiretroviral therapy in low-income and middle-income countries: a systematic review and meta-regression analysis. Lancet Infect. Dis. 2017, 18, 346-355; Zazzi, M., Hu, H., Prosperi, M. The global burden of HIV-1 drug resistance in the past 20 years. PeerJ. 2018, DOI 10.7717/peerj.4848). As a result, new drugs are needed that are easier to take, have high genetic barriers to the development of resistance and have improved safety over current agents. In this panoply of choices, novel mechanisms of action (MOAs) that can be used as part of the preferred antiretroviral therapy (ART) can still have a major role to play since they should be effective against viruses resistant to current agents.

Certain potentially therapeutic compounds have now been described in the art and set forth in Blair, Wade S. et.al. Antimicrobial Agents and Chemotherapy (2009), 53(12), 5080-5087, Blair, Wade S. et al. PLoS Pathogens (2010), 6(12), e1001220, Thenin-Houssier, Suzie; Valente, Susana T. Current HIV Research, 2016, 14, 270-282, and PCT Patent applications with the following numbers: WO 2012065062, WO 2013006738, WO 2013006792, WO 2014110296, WO 2014110297, WO 2014110298, WO 2014134566, WO 2015130964, WO 2015130966, WO 2016033243, WO 2018035359, WO 2018203235, WO 2019161017, and WO 2019161280.

What is now needed in the art are additional compounds which are novel and useful in the treatment of HIV. Additionally, these compounds should provide advantages for pharmaceutical uses, for example, with regard to one or more of their mechanisms of action, binding, inhibition efficacy, target selectivity, solubility, safety profiles, bioavailability or reduced frequency of dosing. Also needed are new formulations and methods of treatment which utilize these compounds. Formulations of certain compounds are disclosed in WO 2020/018459. WO 2020/058844 discloses pyridopyrimidinone-containing compounds for use in the treatment of HIV. WO 2019/198024 discloses capsid inhibitors for the treatment of HIV. WO 2020/157692 discloses a pyridopyrimidinone containing compound for use in the treatment of HIV. WO 2020/254985 discloses a pyridopyrimidinone containing compound for use in the treatment of HIV. "Progress in Medicinal Chemistry", Swallow, S et al., Elsevier, 2015, Chapter 2 discloses the use of fluorine in medicinal chemistry. Glunz, P.W, "Recent encounters with atropisomerism in drug discovery", Bioorganic & Medicinal Chemistry Letters, Volume 28, Issue 2, Elsevier, pages 53-56 discloses atropisomerism in medicinal chemistry.

### SUMMARY OF THE INVENTION

Briefly, in one aspect, the present invention discloses a pharmaceutical composition comprising the compound of Formula Ia, Formula Ib, Formula Ic, or Formula Id: 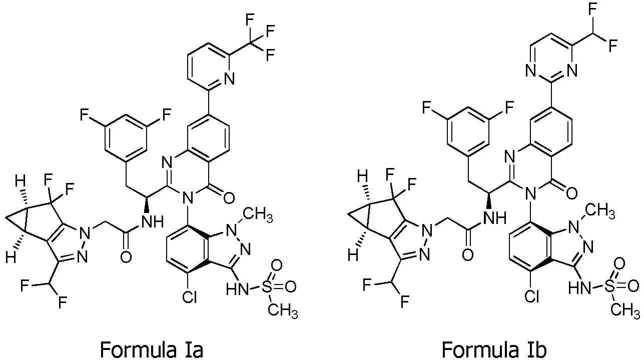 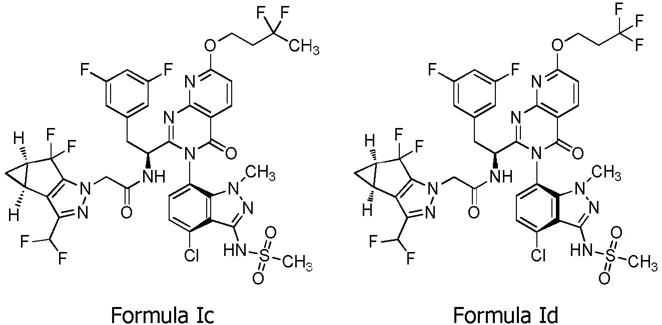 or a pharmaceutically acceptable salt thereof, and a solvent or diluent selected from the group consisting of water, an alcohol, polyethylene glycol (PEG), N-methyl-2-pyrrolidone (NMP), ethyl lactate, propylene glycol, glycofurol, and dimethylsulfoxide (DMSO), wherein the compound of Formula Ia, Formula Ib, Formula Ic, Formula Id, or pharmaceutically acceptable salt thereof, is present in the composition at a concentration of 50 to 500 mg/mL, based on weight of a free compound of Formula Ia, Formula Ib, Formula Ic, Formula Id.

In another aspect, the present invention discloses a pharmaceutical composition of the invention for use in therapy.

In another aspect, the present invention discloses a pharmaceutical composition of the invention for use in treating HIV infection in a human.

### BRIEF DESCRIPTION OF THE FIGURE

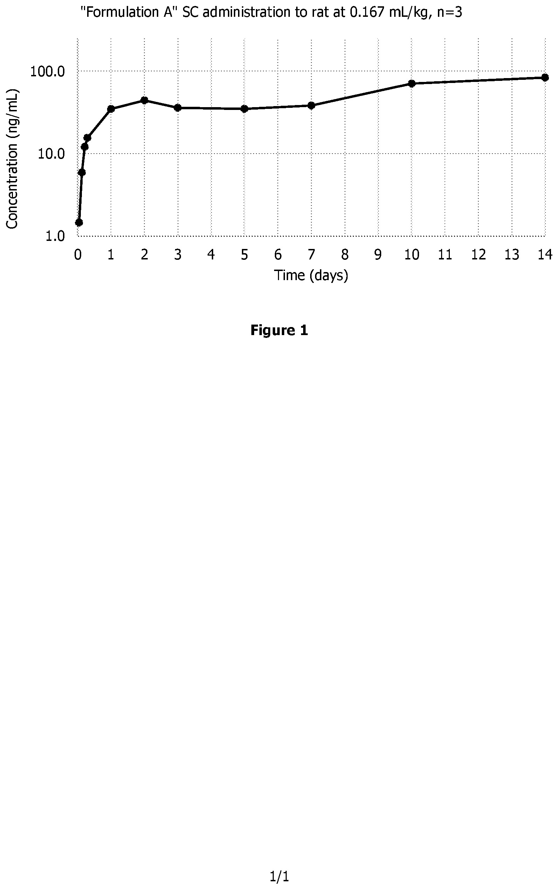

Figure 1 is a summary of the the PK experiment described below.

### DETAILED DESCRIPTION OF THE INVENTION

In one aspect, the pharmaceutical composition of the invention further comprises an excipient selected from the group consisting of polysorbate 20, polysorbate 80, poloxamer 188, poloxamer 207, poloxamer 338, sodium chloride, and sodium hydroxide.

In one aspect, the pharmaceutical composition of the invention is a solution.

In one aspect, the pharmaceutical composition of the invention is a suspension.

In one aspect, the pharmaceutical composition of the invention comprises polyethylene glycol (PEG).

In one aspect, the pharmaceutical composition of the invention comprises polyethylene glycol (PEG). wherein the average molecular weight of PEG is about 300 (PEG 300).

In one aspect, the pharmaceutical composition of the invention comprises ethanol.

In one aspect, the pharmaceutical composition of the invention comprises ethyl lactate.

In one aspect, the pharmaceutical composition of the invention comprises poloxamer 188.

In one aspect, the pharmaceutical composition of the invention comprises sodium hydroxide.

In one aspect, the pharmaceutical composition of the invention is a pharmaceutical composition wherein at least 90 % by weight of the solvents or diluents are water and PEG 300.

In one aspect, the pharmaceutical composition of the invention is a pharmaceutical composition wherein at least 90 % by weight of the excipient is poloxamer 188.

In one aspect, the pharmaceutical composition of the invention is a pharmaceutical composition wherein water is present in the composition at 8 to 20 % by weight and PEG 300 is present in the composition at 60-85 % by weight.

In one aspect, the pharmaceutical composition of the invention is a pharmaceutical composition wherein water is present in the composition at 8 to 12 % by weight and PEG 300 is present in the composition at 63-70 % by weight.

Throughout this specification, reference to concentration of the compound of Formula Ia, Formula Ib, Formula Ic, or Formula Id, or a pharmaceutically acceptable salt thereof, always refer to a concentration based on the free compound of Formula Ia, Formula Ib, Formula Ic, or Formula Id and not on the salt.

In one aspect, the pharmaceutical composition of the invention is a pharmaceutical composition wherein the compound of Formula Ia, Formula Ib, Formula Ic, or Formula Id, or a pharmaceutically acceptable salt thereof, is present in the composition at a concentration of 225 to 275 mg/mL.

In one aspect, the pharmaceutical composition of the invention is a pharmaceutical composition wherein the compound of Formula Ia, Formula Ib, Formula Ic, or Formula Id, or a pharmaceutically acceptable salt thereof, is present in the composition at a concentration of 275 to 350 mg/mL.

In one aspect, the pharmaceutical composition of the invention is a pharmaceutical composition wherein the compound of Formula Ia, Formula Ib, Formula Ic, or Formula Id, or a pharmaceutically acceptable salt thereof, is present in the composition at a concentration of 350 to 425 mg/mL.

In one aspect, the pharmaceutical composition of the invention is produced from a crystalline form of the compound of Formula Ia, Formula Ib, Formula Ic, or Formula Id, or a pharmaceutically acceptable salt thereof. In another aspect, the pharmaceutical composition of the invention is a suspension of a crystalline form of the compound of Formula Ia, Formula Ib, Formula Ic, or Formula Id, or a pharmaceutically acceptable salt thereof.

In one aspect, the pharmaceutical composition of the invention is produced from an amorphous form of the compound of Formula Ia, Formula Ib, Formula Ic, or Formula Id, or a pharmaceutically acceptable salt thereof. In another aspect, the amorphous form is a lyophilized powder. In another aspect, the pharmaceutical composition of the invention is a suspension of an amorphous form.

In another aspect, the formulation comprises water. In another aspect, the formulation comprises glycofurol. In another aspect, the formulation comprises N-methyl-2-pyrrolidone (NMP). In another aspect, the formulation comprises dimethylsulfoxide (DMSO). In another aspect, the formulation comprises an alcohol. In another aspect, the formulation comprises ethanol. In another aspect, the formulation comprises saline. In another aspect, the formulation comprises polyethylene glycol (PEG) for which the chemical formula can generally be written as H-(O-CH₂-CH₂)ₙ-OH. In another aspect, the formulation comprises PEG having an average molecular weight of about 100 (PEG 100). In another aspect, the formulation comprises PEG having an average molecular weight of about 200 (PEG 200). In another aspect, the formulation comprises PEG having an average molecular weight of about 300 (PEG 300). In another aspect, the formulation comprises PEG having an average molecular weight of about 400 (PEG 400). In another aspect, the formulation comprises PEG having an average molecular weight of about 500 (PEG 500). In another aspect, the formulation comprises PEG having an average molecular weight of about 600 (PEG 600). In another aspect, the formulation comprises PEG which is "capped" by an alkyl group such that the formula for PEG can be generally written as alkyl-(O-CH₂-CH₂)ₙ-O-alkyl. In another aspect, the ingredients which comprise >50 w/w% of the formulation are other than water. In another aspect, the ingredients with comprise >60 w/w% of the formulation are other than water. In another aspect, the ingredients with comprise >70 w/w% of the formulation are other than water. In another aspect, the ingredients with comprise >80 w/w% of the formulation are other than water. In another aspect, the ingredients with comprise >85 w/w% of the formulation are other than water. In another aspect, the ingredients with comprise >90 w/w% of the formulation are other than water. In another aspect, the formulation comprises water and PEG 200. In another aspect, the formulation comprises water and PEG 300.

In one aspect, the pharmaceutical composition of the invention comprises the compound of Formula Ia, Formula Ib, Formula Ic, or Formula Id, or a pharmaceutically acceptable salt thereof, and a solubilizing excipient. In another aspect, the solubilizing excipient is polysorbate. In another aspect, the solubilizing excipient is polysorbate 20. In another aspect, the solubilizing excipient is polysorbate 80. In another aspect, the solubilizing excipient is poloxamer. In another aspect, the solubilizing excipient is poloxamer 188 (P188). In another aspect, the solubilizing excipient is poloxamer 207. In another aspect, the solubilizing excipient is poloxamer 338 (P338). In another aspect, the solubilizing excipient is docusate sodium. In another aspect, the solubilizing excipient is polyethylene glycol 3350 (PEG3350). In another aspect, the solubilizing excipient is a fatty acid. In another aspect, the solubilizing excipient is a fatty acid comprising 8-12 carbons. In another aspect, the solubilizing excipient is a fatty acid comprising 8 carbons (C8). In another aspect, the solubilizing excipient is a fatty acid comprising 12 carbons (C12). In another aspect, the solubilizing excipient is less than 5 w/w% of the formulation. In another aspect, the solubilizing excipient is less than about 5 w/w% of the formulation. In another aspect, the solubilizing excipient is less than about 4 w/w% of the formulation. In another aspect, the solubilizing excipient is less than about 3 w/w% of the formulation. In another aspect, the solubilizing excipient is less than about 2 w/w% of the formulation. In another aspect, the solubilizing excipient is less than about 1 w/w% of the formulation. In another aspect, the solubilizing excipient is less than about 0.5 w/w% of the formulation. In another aspect, the solubilizing excipient is less than about 0.25 w/w% of the formulation.

In one aspect, the pharmaceutical composition of the invention comprises poloxamer 188 and a C8 fatty acid. In another aspect, the ratio of poloxamer 188 and the C8 fatty acid is about 3:1. In another aspect, the concentration of poloxamer 188 is about 3 w/w% of the formulation and the concentration of the C8 fatty acid is about 1 w/w% of the formulation.

In one aspect, the pharmaceutical composition of the invention comprises poloxamer 188 and a C12 fatty acid. In another aspect, the ratio of poloxamer 188 and the C12 fatty acid is about 6:1. In another aspect, the concentration of poloxamer 188 is about 3 w/w% of the formulation and the concentration of the C12 fatty acid is about 0.5 w/w% of the formulation.

In one aspect, the pharmaceutical composition of the invention comprises poloxamer 338 and a C8 fatty acid. In another aspect, the ratio of poloxamer 338 and the C8 fatty acid is about 3:1. In another aspect, the concentration of poloxamer 388 is about 3 w/w% of the formulation and the concentration of the C8 fatty acid is about 1 w/w% of the formulation.

In one aspect, the pharmaceutical composition of the invention comprises poloxamer 338 and a C12 fatty acid. In another aspect, the ratio of poloxamer 388 and the C12 fatty acid is about 6:1. In another aspect, the concentration of poloxamer 388 is about 3 w/w% of the formulation and the concentration of the C12 fatty acid is about 0.5 w/w% of the formulation.

In one aspect, the pharmaceutical composition of the invention comprises docusate sodium and a C8 fatty acid. In another aspect, the ratio of docusate sodium and the C8 fatty acid is about 3:1. In another aspect, the concentration of docusate sodium is about 3 w/w% of the formulation and the concentration of the C8 fatty acid is about 1 w/w% of the formulation.

In one aspect, the pharmaceutical composition of the invention comprises docusate sodium and a C12 fatty acid. In another aspect, the ratio of docusate sodium and the C12 fatty acid is about 6:1. In another aspect, the concentration of docusate sodium is about 3 w/w% of the formulation and the concentration of the C12 fatty acid is about 0.5 w/w% of the formulation.

In one aspect, the pharmaceutical composition of the invention contains a chelating agent. In another aspect, the chelating agent is Na-EDTA. In another aspect, Na-EDTA is present as 0.01-0.05 w/w% of the formulation.

In one aspect, the pharmaceutical composition of the invention contains an antioxidant. In another aspect, the antioxidant is L-methionine. In another aspect, the antioxidant is vitamin E. In another aspect, the antioxidant is present as 0.01-0.10 w/w% of the formulation.

In one aspect, the pharmaceutical composition of the invention contains an acid. In another aspect, the acid is ethanesulfonic acid. In another aspect, the concentration of ethanesulfonic acid is 1-10 mM. In another aspect, the concentration of ethanesulfonic acid is 4.8-5.2 mM. In another aspect, the acid is methanesulfonic acid. In another aspect, the concentration of methanesulfonic acid is 1-10 mM. In another aspect, the concentration of methanesulfonic acid is 4.8-5.2 mM.

In one aspect, the pharmaceutical composition of the invention contains a base. In another aspect, the base is an inorganic base. In another aspect, the base is an organic base. In another aspect, the anion of the base is ⁻OH (hydroxide). In another aspect, the anion of the base is ⁻OEt (ethoxide). In another aspect, the anion of the base is ⁻OAc (acetate). In another aspect, the cation of the base is ⁺Na (sodium). In another aspect, the cation of the base is ⁺K (potassium). In another aspect, the cation of the base is choline. In another aspect, the base is sodium acetate. In another aspect, the base is sodium hydroxide. In another aspect, the base is sodium ethoxide. In another aspect, the base is choline hydroxide. In another aspect, the pharmaceutical composition of the invention comprises 0.1-1.5 molar equivalents of base relative to the compound of Formula Ia, Formula Ib, Formula Ic, or Formula Id. In another aspect, the pharmaceutical composition of the invention comprises 0.7-1.2 molar equivalents of base relative to the compound of Formula Ia, Formula Ib, Formula Ic, or Formula Id. In another aspect, the pharmaceutical composition of the invention comprises 0.7-1.0 molar equivalents of base relative to the compound of Formula Ia, Formula Ib, Formula Ic, or Formula Id. In another aspect, the pharmaceutical composition of the invention comprises 1.0-1.2 molar equivalents of base relative to the compound of Formula Ia, Formula Ib, Formula Ic, or Formula Id. In another aspect, the pharmaceutical composition of the invention comprises about equal molar equivalents of base and the compound of Formula Ia, Formula Ib, Formula Ic, or Formula Id.

In one aspect, the pharmaceutical composition of the invention comprises a buffer. In another aspect, the buffer is an acetate buffer. In another aspect, the pH of the buffer is 4.0-7.4. In another aspect, the pH of the buffer is 4.0-5.5. In another aspect, the pH of the buffer is 4.8-5.2. In another aspect, the concentration of the buffer is 1-10 mM. In another aspect, the concentration of the buffer is 4.8-5.2 mM.

In one aspect, the pharmaceutical composition of the invention comprises 50-500 mg/mL of the compound of Formula Ia, Formula Ib, Formula Ic, or Formula Id, or a pharmaceutically acceptable salt thereof. In another aspect, the pharmaceutical composition of the invention comprises about 50 mg/mL of the compound of Formula Ia, Formula Ib, Formula Ic, or Formula Id, or a pharmaceutically acceptable salt thereof. In another aspect, the pharmaceutical composition of the invention comprises about 100 mg/mL of the compound of Formula Ia, Formula Ib, Formula Ic, or Formula Id, or a pharmaceutically acceptable salt thereof. In another aspect, the pharmaceutical composition of the invention comprises about 150 mg/mL of the compound of Formula Ia, Formula Ib, Formula Ic, or Formula Id, or a pharmaceutically acceptable salt thereof. In another aspect, the pharmaceutical composition of the invention comprises about 200 mg/mL of the compound of Formula Ia, Formula Ib, Formula Ic, or Formula Id, or a pharmaceutically acceptable salt thereof. In another aspect, the pharmaceutical composition of the invention comprises about 250 mg/mL of the compound of Formula Ia, Formula Ib, Formula Ic, or Formula Id, or a pharmaceutically acceptable salt thereof. In another aspect, the pharmaceutical composition of the invention comprises about 300 mg/mL of the compound of Formula Ia, Formula Ib, Formula Ic, or Formula Id, or a pharmaceutically acceptable salt thereof. In another aspect, the pharmaceutical composition of the invention comprises about 350 mg/mL of the compound of Formula Ia, Formula Ib, Formula Ic, or Formula Id, or a pharmaceutically acceptable salt thereof. In another aspect, the pharmaceutical composition of the invention comprises about 400 mg/mL of the compound of Formula Ia, Formula Ib, Formula Ic, or Formula Id, or a pharmaceutically acceptable salt thereof. In another aspect, the pharmaceutical composition of the invention comprises about 450 mg/mL of the compound of Formula Ia, Formula Ib, Formula Ic, or Formula Id, or a pharmaceutically acceptable salt thereof. In another aspect, the pharmaceutical composition of the invention comprises about 500 mg/mL of the compound of Formula Ia, Formula Ib, Formula Ic, or Formula Id, or a pharmaceutically acceptable salt thereof.

In one aspect, the pharmaceutical composition of the invention comprises the compound of Formula Ia, Formula Ib, Formula Ic, or Formula Id, or a pharmaceutically acceptable salt thereof, water, PEG 300, and 0-3 additional excipients.

In one aspect, the pharmaceutical composition of the invention comprises the compound of Formula Ia, Formula Ib, Formula Ic, or Formula Id, or a pharmaceutically acceptable salt thereof, water, PEG 200, and 0-3 additional excipients.

In one aspect, the pharmaceutical composition of the invention comprises the compound of Formula Ia, Formula Ib, Formula Ic, or Formula Id, or a pharmaceutically acceptable salt thereof, poloxamer P338, and acetate buffer. In another aspect, the concentration of Formula Ia, Formula Ib, Formula Ic, or Formula Id, or a pharmaceutically acceptable salt thereof is 280-320 mg/mL. In another aspect, the concentration of poloxamer P338 is 3-5 w/w%. In another aspect, the pH of the acetate buffer is 4.8-5.2. In another aspect, the concentration of the acetate buffer is 4.8-5.2 mM.

In one aspect, the pharmaceutical composition of the invention comprises the compound of Formula Ia, Formula Ib, Formula Ic, or Formula Id, or a pharmaceutically acceptable salt thereof, poloxamer P338, ethanesulfonic acid, and acetate buffer. In another aspect, the concentration of Formula Ia, Formula Ib, Formula Ic, or Formula Id, or a pharmaceutically acceptable salt thereof is 280-320 mg/mL. In another aspect, the concentration of poloxamer P338 is 3-5 w/w%. In another aspect, the concentration of ethanesulfonic acid is 4.8-5.2 mM. In another aspect, the pH of the acetate buffer is 4.8-5.2. In another aspect, the concentration of the acetate buffer is 4.8-5.2 mM.

In one aspect, the pharmaceutical composition of the invention comprises the compound of Formula Ia, Formula Ib, Formula Ic, or Formula Id, or a pharmaceutically acceptable salt thereof, poloxamer P338, a C8-C12 fatty acid, and acetate buffer. In another aspect, the concentration of Formula Ia, Formula Ib, Formula Ic, or Formula Id, or a pharmaceutically acceptable salt thereof is 280-320 mg/mL. In another aspect, the concentration of poloxamer P338 is 3-5 w/w%. In another aspect the concentration of the fatty acid is 0.5-1 w/w%. In another aspect, the pH of the acetate buffer is 4.8-5.2. In another aspect, the concentration of the acetate buffer is 4.8-5.2 mM.

In one aspect, the pharmaceutical composition of the invention comprises the compound of Formula Ia, Formula Ib, Formula Ic, or Formula Id, or a pharmaceutically acceptable salt thereof, docusate sodium, and acetate buffer. In another aspect, the concentration of Formula Ia, Formula Ib, Formula Ic, or Formula Id, or a pharmaceutically acceptable salt thereof is 280-320 mg/mL. In another aspect, the concentration of docusate sodium is 1-2 w/w%. In another aspect, the pH of the acetate buffer is 4.8-5.2. In another aspect, the concentration of the acetate buffer is 4.8-5.2 mM.

In one aspect, the present invention discloses a pharmaceutical composition which is a solution, and which is rendered sterile by passage through a filter.

The salts of the invention are pharmaceutically acceptable. Such salts may be acid addition salts or base addition salts. For a review of suitable pharmaceutically acceptable salts see, for example, Berge et al, J. Pharm, Sci., 66, 1-19, 1977.

Representative pharmaceutically acceptable acid addition salts include, but are not limited to, 4-acetamidobenzoate, acetate, adipate, alginate, ascorbate, aspartate, benzenesulfonate (besylate), benzoate, bisulfate, bitartrate, butyrate, calcium edetate, camphorate, camphorsulfonate (camsylate), caprate (decanoate), caproate (hexanoate), caprylate (octanoate), cinnamate, citrate, cyclamate, digluconate, 2,5-dihydroxybenzoate, disuccinate, dodecylsulfate (estolate), edetate (ethylenediaminetetraacetate), estolate (lauryl sulfate), ethane-1,2-disulfonate (edisylate), ethanesulfonate (esylate), formate, fumarate, galactarate (mucate), gentisate (2,5-dihydroxybenzoate), glucoheptonate (gluceptate), gluconate, glucuronate, glutamate, glutarate, glycerophosphorate, glycolate, hexylresorcinate, hippurate, hydrabamine (*N*,*N*'-di(dehydroabietyl)-ethylenediamine), hydrobromide, hydrochloride, hydroiodide, hydroxynaphthoate, isobutyrate, lactate, lactobionate, laurate, malate, maleate, malonate, mandelate, methanesulfonate (mesylate), methylsulfate, mucate, naphthalene-1,5-disulfonate (napadisylate), naphthalene-2-sulfonate (napsylate), nicotinate, nitrate, oleate, palmitate, *p*-aminobenzenesulfonate, *p*-aminosalicyclate, pamoate (embonate), pantothenate, pectinate, persulfate, phenylacetate, phenylethylbarbiturate, phosphate, polygalacturonate, propionate, *p*-toluenesulfonate (tosylate), pyroglutamate, pyruvate, salicylate, sebacate, stearate, subacetate, succinate, sulfamate, sulfate, tannate, tartrate, teoclate (8-chlorotheophyllinate), thiocyanate, triethiodide, undecanoate, undecylenate, and valerate.

Representative pharmaceutically acceptable base addition salts include, but are not limited to, aluminium, 2-amino-2-(hydroxymethyl)-1,3-propanediol (TRIS, tromethamine), arginine, benethamine (*N-*benzylphenethylamine), benzathine (*N,N*'-dibenzylethylenediamine), *bis*-(2-hydroxyethyl)amine, bismuth, calcium, chloroprocaine, choline, clemizole (1-*p* chlorobenzyl-2-pyrrolildine-1-ylmethylbenzimidazole), cyclohexylamine, dibenzylethylenediamine, diethylamine, diethyltriamine, dimethylamine, dimethylethanolamine, dopamine, ethanolamine, ethylenediamine, L-histidine, iron, isoquinoline, lepidine, lithium, lysine, magnesium, meglumine (*N-*methylglucamine), piperazine, piperidine, potassium, procaine, quinine, quinoline, sodium, strontium, *t*-butylamine, and zinc.

A particularly preferred salt is a sodium salt. Another particularly preferred salt is a potassium salt.

In another aspect the present invention discloses methods of preventing HIV infection in a human or reducing the risk of infection, comprising administering a pharmaceutical composition of the invention. Pre-exposure prophylaxis (or PrEP) is when people at risk for HIV infection take daily medicine to lower their chances of getting HIV infection. PrEP has been shown to be effective in reducing the risk of infection.

The compounds and salts of this invention are believed to have as their biological target the HIV capsid and thus their mechanism of action is to modify in one or more ways the function of the HIV capsid.

The compounds and salts of the present invention may be employed alone or in combination with other therapeutic agents. Combination therapies according to the present invention thus comprise the administration of at least one compound or salt of the invention, and the administration of at least one other agent which may be useful in the treatment of HIV infection. A compound or salt of the present invention, and the other agent may be formulated and administered together in a single pharmaceutical composition or may be formulated and administered separately. When formulated and administered separately, administration may occur simultaneously or sequentially in any order. Suitable other agents include, for example, abacavir, atazanavir, bictegravir, cabotegravir, darunavir, delavirdine, didanosine, dideoxyinosine, dolutegravir, doravirine, efavirenz, elvitegravir, emtricitabine, etavirine, fosamprenavir, fostemsavir, indinavir, slatravir, lamivudine, lopinavir, maraviroc, nelfinavir, nevirapine, raltegravir, rilpiverine, ritonavir, saquinavir, stavudine, tipranavir, tenofovir, tenofovir alafenamide, tenofovir disoproxil fumarate, zalcitabine, and zidovudine. Preferred agents include, for example, dolutegravir, bictegravir, islatravir, lamivudine, fostemsavir, and cabotegravir. Particularly preferred agents include, for example, dolutegravir, bictegravir, lamivudine, fostemsavir, and cabotegravir.

### EXAMPLES

### Preparation of bicyclo[3.1. 0]hexan-3-ol

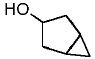

To a stirred solution of cyclopent-3-enol (130 g, 1545 mmol) in DCM (1200 mL) under N₂ atmosphere at 0-5 °C was added dropwise a solution of diethyl zinc in hexane (1.0 M, 3091 mL, 3091 mmol) over a period of 3 h. To the solution at 0 °C was added dropwise a solution of diiodomethane (249 mL, 3091 mmol) in DCM (300 mL) over a period of 1h. The reaction mixture was allowed to warm to 27 °C upon which formation of a white precipitation was observed. The mixture stirred for 16 h. Progress of the reaction was monitored by TLC (SiO₂, 20% EtOAc/pet, Rf = 0.3, UV-inactive, PMA-active). The reaction mixture was quenched via the careful addition of aq. saturated NH₄Cl solution (1.5 L). The mixture was filtered through pad of Celite. The aqueous layer was extracted with DCM (2 x 1L). The combined organic layers were dried over anhydrous Na₂SO₄, filtered and then concentrated under reduced pressure to afford crude bicyclo[3.1.0]hexan-3-ol as red liquid, 180 g. ¹H NMR (400 MHz, CDCl₃) δ = 4.41 - 4.35 (m, 1H), 2.18 - 2.05 (m, 2H), 1.73 (d, *J=* 13.9 Hz, 2H), 1.35 - 1.25 (m, 2H), 1.21 - 1.14 (m, 1H), 0.57 - 0.43 (m, 2H). GCMS: m/z = 98.1).

### Preparation of bicyclo[3.1.O]hexan-3-one

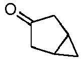

To a stirred solution of bicyclo[3.1.0]hexan-3-ol (210 g, 2054 mmol) in DCM (5000 mL) under N₂ atmosphere at 0 °C was added portion-wise Dess-Martin periodinane (954 g, 225 mmol). The mixture was allowed to warm to 27 °C and was then stirred for 16 h. Progress of the reaction was monitored by TLC (SiO₂, 20% Acetone/Hex, Rf = 0.3, UV inactive, PMA-active). The reaction mixture was filtered through pad of Celite and the filtrate was washed with aq. NaOH (1N, 8x 1 L). The combined aqueous phases were extracted with DCM (5 X 1 L). The combined organic layers were dried over anhydrous Na₂SO₄, filtered, and then concentrated under reduced pressure (bath temperature: 20 °C) to afford crude bicyclo[3.1.0]hexan-3-one as brown liquid. The liquid was further purified by downward distillation at 70 °C to afford bicyclo[3.1.0]hexan-3-one as a pale-yellow viscous liquid, 125 g (62%). ¹H NMR (400 MHz, CDCl₃) δ = 2.61 - 2.54 (m, 2H), 2.17 - 2.12 (m, 2H), 1.54 - 1.46 (m, 2H), 0.92 - 0.86 (m, 1H), -0.01 - -0.08 (m, 1H); GCMS: M/Z = 96.1.

### Preparation of 2-(2,2-difluoroacetyl)bicyclo[3.1.0]hexan-3-one

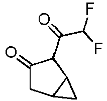

To a stirred solution of bicyclo[3.1.0]hexan-3-one (125 g, 1274 mmol) in THF (1500 mL) under N₂ atmosphere at -78 °C was added LDA (2.0 M in THF, 0.701 L, 1402 mmol). The solution was stirred for 1 h at -78 °C. To the solution was added slowly over 30 minutes a solution of ethyldifluoroacetate (174 g, 1402 mmol) in THF (300 mL) maintaining a temperature of -78 °C. The reaction mixture was allowed to warm to 27 °C and was then stirred for 1 h. Progress of the reaction was monitored by TLC (SiO₂, 20% Acetone/Hexane, Rf = 0.3, UV -active). The reaction mixture was quenched via the addition of aq. HCl (1N, 2000 mL). The mixture was stirred for 30 min. and then was extracted with EtOAc (3 x 1000 mL). The combined organic layers were washed with brine (1000 mL), dried over anhydrous Na₂SO₄ and filtered. The filtrate was concentrated under reduced pressure to afford 2-(2,2-difluoroacetyl)bicyclo[3.1.0]hexan-3-one as a pale-yellow viscous liquid, 180 g (71%). ¹H NMR (400 MHz, CDCl₃) δ = 6.18 (t, *J* = 54.8 Hz, 1H), 2.70 - 2.62 (m, 1H), 2.35 (d, *J* = 19.4 Hz, 1H), 2.14 (br s, 1H), 1.26 - 1.21 (m, 1H), 1.04-1.03 (m, 1H), 0.22-0.21 (m, 1H), LCMS: M/Z = 173.17).

### Preparation of ethyl 2-(3-(difluoromethyl)-3b,4,4a,5-tetrahydro-1H-cyclopropa[3,4]cyclopenta[1,2-c]pyrazol-1-yl)acetate.

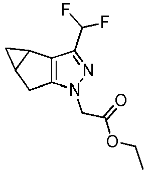

To a stirred solution of 2-(2,2-difluoroacetyl)bicyclo[3.1.0]hexan-3-one (180 g, 910 mmol) in ethanol (2 L) under N₂ atmosphere at 27 °C was added ethyl 2-hydrazinylacetate hydrochloride (422 g, 2729 mmol) followed by sulfuric acid (20 mL, 375 mmol). The mixture was stirred for 30 min. and then was heated to 100 °C and stirred for 16 h. Progress of the reaction was monitored by TLC (SiO₂, 20% Acetone/Hexane, Rf = 0.3, UV-active). The reaction mixture was concentrated under reduced pressure. The residue was dissolved in EtOAc (2000 mL) and was washed with water (2 x 1 L), brine (1.0 L), dried over anhydrous Na₂SO₄, filtered, and then was concentrated under reduced pressure. The resulting residue was subjected to silica gel column chromatography (pet.:acetone 100:0→98:2) to afford ethyl 2-(3-(difluoromethyl)-3b,4,4a,5-tetrahydro-1H-cyclopropa[3,4]cyclopenta[1,2-c]pyrazol-1-yl)acetate as an off-white solid, 110 g (46%). ¹H NMR (400 MHz, DMSO-d₆) δ = 6.86 (t, *J=* 54.8 Hz, 1H), 4.93 (s, 2H), 4.14 (q, *J=* 7.2 Hz, 2H), 2.88 - 2.79 (m, 1H), 2.76 - 2.68 (m, 1H), 2.14 - 2.04 (m, 2H), 1.19 (t, *J=* 7.2 Hz, 3H), 1.10 - 1.03 (m, 1H), 0.14 (q, *J* = 4.3 Hz, 1H).

### Preparation of ethyl 2-(3-(difluoromethyl)-5-oxo-3b,4,4a,5-tetrahydro-1H-cyclopropa[3,4]cyclopenta[1,2-c]pyrazol-1-yl)acetate.

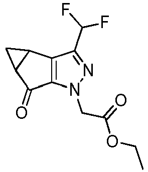

To a stirred solution of ethyl 2-(3-(difluoromethyl)-3b,4,4a,5-tetrahydro-1H-cyclopropa[3,4]cyclopenta[1,2-c]pyrazol-1-yl)acetate (110 g, 422 mmol) and Celite (395 g) in cyclohexane (3.5 L) at 0 °C was added portionwise pyridinium dichromate (794 g, 2110 mmol). To the mixture under nitrogen atmosphere was added dropwise tert-butyl hydroperoxide (355 mL, 2130 mmol) over a period of 10 min. The reaction mixture was warmed to 27 °C and was then stirred at that temperature for 48 h. Progress of the reaction was monitored by TLC (SiO₂, 30% Acetone/pet, Rf = 0.4, UV -active). The reaction mixture was filtered and the filter cake was extracted with EtOAc (1000 mL). The filtrate was washed with saturated aq. Na₂S₂O₃ (2x500 mL); saturated aq. FeSO₄ (300 mL); and then brine (500 mL). The organic layer was dried over anhydrous Na₂SO₄, filtered and concentrated under reduced pressure to obtain the crude title compound (150 g).

### Preparation of ethyl 2-(3-(difluoromethyl)-4,4a-dihydrospiro[cyclopropa[3,4]cyclopenta[1,2-c]pyrazole-5,2'-[1,3]dithiolane]-1(3bH)-yl)acetate.

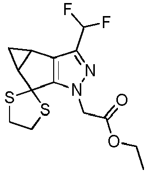

To a stirred solution of ethyl 2-(3-(difluoromethyl)-5-oxo-3b,4,4a,5-tetrahydro-1H-cyclopropa[3,4]cyclopenta[1,2-c]pyrazol-1-yl)acetate (75 g, 269 mmol) in DCM (1500 mL) at 27 °C under nitrogen atmosphere was added ethane-1,2-dithiol (43.0 mL, 511 mmol) followed by the addition of boron trifluoride acetic acid (72.6 mL, 511 mmol). The solution was stirred for 16 h. Progress of the reaction was monitored by TLC (SiO₂, 20% Acetone/Pet, Rf = 0.35, UV -Active). After completion, the reaction mixture was cooled to 0 °C and quenched via the addition of aq. saturated NaHCO₃ (500 mL). The mixture was extracted with DCM (2 X 1000 mL). The combined organics were washed with brine (1000 mL), dried over anhydrous Na₂SO₄, filtered and concentrated under reduced pressure to obtain a brown liquid. This material was subjected to silica gel column chromatography (Pet.:EtOAc 95:5→90:10) to afford ethyl 2-(3-(difluoromethyl)-4,4a-dihydrospiro[cyclopropa[3,4]cyclopenta[1,2-c]pyrazole-5,2'-[1,3]dithiolane]-1(3bH)-yl)acetate as an off-white solid, 80 g (74%). ¹H-NMR (400 MHz, CDCl₃) δ = 6.61 (t, *J=* 55.2 Hz, 1H), 5.00 - 4.85 (m, 2H), 4.29 - 4.19 (m, 2H), 3.55 - 3.46 (m, 4H), 2.63 - 2.53 (m, 1H), 2.49 - 2.38 (m, 1H), 1.30 - 1.24 (m, 4H), 0.65 - 0.60 (m, 1H). LCMS M+H = 346.9.

### Preparation of ethyl 2-(3-(difluoromethyl)-5,5-difluoro-3b,4,4a,5-tetrahydro-1H-cyclopropa[3,4]cyclopenta[1,2-c]pyrazol-1-yl)acetate

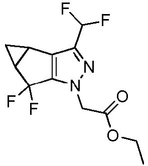

To a stirred solution of 1,3-dibromo-5,5-dimethylimidazolidine-2,4-dione (26.3 g, 92 mmol) in DCM (20 mL) at -70 °C under N₂ atmosphere was added HF-pyridine (2.460 g, 24.83 mmol). The solution was for 30 min. To the solution was added a solution of ethyl 2-(3-(difluoromethyl)-4,4a-dihydrospiro[cyclopropa[3,4]cyclopenta[1,2-c]pyrazole-5,2'-1,3]dithiolane]-1(3bH)-yl)acetate (10 g, 25 mmol) in DCM (20 mL). The reaction mixture was allowed to warm to -40 °C and then was stirred at that temperature for 1 h. Progress of the reaction was monitored by TLC (SiO2, 30% EtOAc/Pet, Rf = 0.3, UV in-active). The reaction mixture was quenched via the addition of aq. sat. NaHCO₃ (200 mL). The mixture was warmed to room temperature and was then extracted with EtOAc (2 x 100 mL). The combined organics were washed with brine (50 mL); dried over anhydrous Na₂SO₄; filtered; and were concentrated under reduced pressure to afford a brown solid. This material was subjected to silica gel column chromatography (Pet.:EtOAc 100:0→75-25) to afford ethyl 2-(3-(difluoromethyl)-5,5-difluoro-3b,4,4a,5-tetrahydro-1H-cyclopropa[3,4]cyclopenta[1,2-c]pyrazol-1-yl)acetate as a pale-yellow solid, 8.5 g (91%). ¹H NMR (400 MHz, CDCl₃) δ = 6.62 (t, J = 55.2 Hz, 1H), 4.82 (s, 2H), 4.30 - 4.18 (m, 2H), 2.51 - 2.37 (m, 2H), 1.42 - 1.35 (m, 1H), 1.31 - 1.23 (m, 3H), 1.14 - 1.08 (m, 1H). LCMS M+H = 293.07.

### Preparation of 2-(3-(difluoromethyl)-5,5-difluoro-3b,4,4a,5-tetrahydro-1H-cyclopropa[3,4]cyclopenta[1,2-c]pyrazol-1-yl)acetic acid

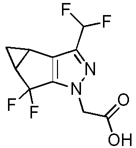

To a stirred solution of ethyl 2-(3-(difluoromethyl)-5,5-difluoro-3b,4,4a,5-tetrahydro-1H-cyclopropa[3,4]cyclopenta[1,2-c]pyrazol-1-yl)acetate (15 g, 50 mmol) in THF (17 mL) and MeOH (66 mL) at 0 °C under N₂ atmosphere was added a solution of LiOH (1.788 g, 74.7 mmol) in water (66 mL). The reaction mixture was allowed to warm to 27 °C and was then stirred for 3 h at that temperature. Progress of the reaction was monitored by TLC (SiO₂, 5% MeOH/DCM, Rf = 0.2, UV Active). After completion, the reaction mixture was concentrated under reduced pressure; diluted with water (50 mL); and washed with EtOAc (2 x 250 mL) to remove impurities. The aqueous layer was adjusted to pH 2-3 using aq. HCl (1M), then was extracted with EtOAc (3 x 1000 mL). The combined organics were dried over anhydrous Na₂SO₄; filtered; and concentrated under reduced pressure to afford 2-(3-(difluoromethyl)-5,5-difluoro-3b,4,4a,5-tetrahydro-1H-cyclopropa[3,4]cyclopenta[1,2-c]pyrazol-1-yl)acetic acid as an off white solid, 14 g (98%). LCMS M+H = 265.15.

### Separation affording 2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetic acid and 2-((3bR,4aS)-3-(difluoromethyl)-5,5-difluoro-3b,4,4a,5-tetrahydro-1H-cyclopropa[3,4]cyclopenta[1,2-c]pyrazol-1-yl)acetic acid

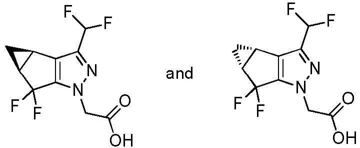

2-(3-(difluoromethyl)-5,5-difluoro-3b,4,4a,5-tetrahydro-1H-cyclopropa[3,4]cyclopenta[1,2-c]pyrazol-1-yl)acetic acid (5.5 g) was dissolved in isopropanol (20 mL). The solution was subjected portion-wise to SFC chiral separation as follows: Instrument = Thar 80; column = Chiralpak IC 30x250mm, 5 micron; solvent A = super critical CO₂; solvent B = isopropanol with 0.5% isopropylamine (v/v); eluent composition = 70%A:30%B; flow-rate = 65 g/min; back-pressure = 100 bar; temperature = 30 °C; injection volume = 2.5 mL; detection = 220 nm. 2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetic acid was collected as peak eluting from 7.5 min. to 14 min; 2-((3bR,4aS)-3-(difluoromethyl)-5,5-difluoro-3b,4,4a,5-tetrahydro-1H-cyclopropa[3,4]cyclopenta[1,2-c]pyrazol-1-yl)acetic acid was collected as a peak eluting from 2.7 min. to 5.8 min. For each enantiomer, the resulting solution was concentrated under reduced pressure and the resulting solids were dissolved in EtOAc, then twice washed with aq. citric acid (1M) followed by water followed by brine. The organic solution was dried over Na₂SO₄; filtered; then concentrated in vacuo to afford the separated enantiomer in 80-90% recovery.

### Preparation of N-(7-amino-4-chloro-1-methyl-1H-indazol-3-yl)-N-(4-methoxybenzyl)methanesulfonamide.

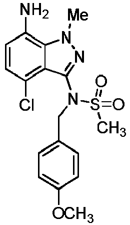

### Synthesis Scheme:

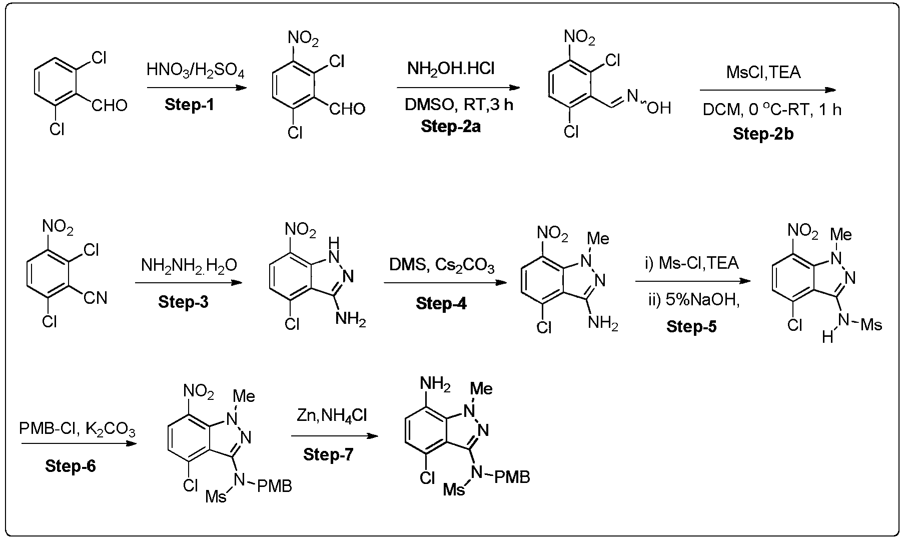

### Step 1: Preparation of 2,6-dichloro-3-nitrobenzaldehyde

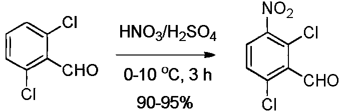

To a solution of sulfuric acid (H₂SO₄) (5.63 L, 4.5 V) in a round-bottom flask at 0-5 °C was added 2,6-dichlorobenzaldehyde (1.25 kg, 7.10 mol, 1.0 equiv.) in portions at below 15 °C. The reaction mass was stirred at 0-5 °C for 30 min. A solution of freshly prepared nitration mixture [Prepared from Conc. H₂SO₄ (0.425 L, 0.34 V) and 70% HNO₃ (0.85 kg, 13.49 mol, 1.30 equiv.) at 0 °C] was added to the above reaction mixture at below 10 °C **[Note:** Reaction is slightly exothermic (3-6 °C); so that addition is preferred at lower temperature]. The reaction mixture was stirred at 5-10 °C for 2-3 h. After completion of the reaction (monitored by TLC), it was quenched with ice cold water (18.75 L, 15 V) at below 25 °C. Then the reaction mass was allowed warm to room temperature and stirred for 2 h. The solids were isolated by filtration and then were washed with water (2.5 L, 2.0 V). Bulk residual water was removed from the solids by maintaining vacuum filtration for 60-90 min. The crude wet solid was initially dried under air atmosphere; then in a hot air oven at 50-55 °C for 10-12 h (until moisture content is not more than 5.0 %) to get the dried title product, 2,6-dichloro-3-nitrobenzaldehyde (1.44 kg, 92% yield) as a yellow solid. ¹H NMR (400 MHz, CDCl₃): *δ* 10. 44 (s, 1H), 7.88 (d, *J* = 8.4 Hz, 1H), 7.56 (d, *J* = 8.8 Hz, 1H).

### Step 2: Preparation of 2,6-dichloro-3-nitrobenzonitrile

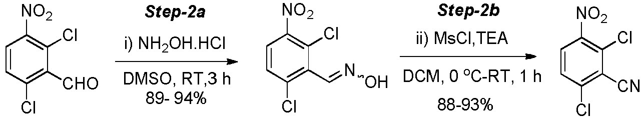

(Step-2a) To a solution of DMSO (5.9 L, 5.0 V)) in a round-bottom flask was added 2,6-dichloro-3-nitrobenzaldehyde (1.17 kg, 5.31 mol, 1.0 equiv.) at room temperature. After being stirred for 30 min at room temperature, hydroxylamine hydrochloride (0.63 kg, 9.04 mol, 1.70 equiv.) was added and the reaction mass was stirred at room temperature for 3 h. After completion of the reaction (monitored by TLC), the reaction mass was quenched by the addition of ice-cold water (18.0 L, 15.0 V) added at a rate sufficient to maintain the temperature below 30 °C (Observation: Solids formed upon water addition). The reaction mass was stirred at room temperature for 60-90 min. The solids were isolated by filtration; washed with water (2.5 L, 2.0 V); followed by washing with a mixture of acetone and hexanes (6.0 L, 1:1 ratio). Bulk residual water was removed from the solids by maintaining vacuum filtration for 60-90 min. The wet solid was initially air dried and then finally dried in a hot air oven at 50-55 °C for 10-12 h (until moisture content was not more than 1.0 %) to get the dried target product, 2,6-dichloro-3-nitrobenzaldehyde oxime (1.22 kg, 92% yield) as an off-white solid. The crude product (which contains 10-20% of 2,6-dichloro-3-nitrobenzonitrile) was used directly in the next step without further purification.
(Step-2b) To a stirred solution of the crude oxime (preparation described above, 1.13 kg, 4.80 mol, 1.0 equiv.) in DCM (9.04 L, 8.0 V) at 0-5 °C was added triethylamine ("TEA", 1.02 kg, 10.09 mol, 2.1 equiv.). After being stirred for 5 min, methanesulfonyl chloride (0.60 kg, 5.29 mol, 1.1 equiv.) was added (Observation: An exotherm is noted during the addition) slowly at 15 °C. Then the reaction mass was stirred at room temperature for 30-45 min. After completion of the reaction (progress of reaction was monitored by TLC; mobile phase: 20% ethyl acetate in hexanes), the reaction mass was diluted with water (6.78 L, 6.0 V); the organic layer was separated; and the aqueous layer was extracted with DCM (3.4 L, 3.0 V). The combined organic layers were washed with brine (5.65 L, 5.0 V); dried over Na₂SO₄; and concentrated under vacuum. The resulting crude solids were triturated with hexanes (4.50 L, 4.0 V) at room temperature. The wet material was dried in a hot air oven at 50-55 °C for 5- 6 h to get the dried product, 2,6-dichloro-3-nitrobenzonitrile (0.95 kg, 91% yield) as a yellow solid. ¹H NMR (400 MHz, CDCl₃): *δ*8.07 (d, *J=* 8.8 Hz, 1H), 7.63 (d, *J* = 8.8 Hz, 1H).

### Step 3: Preparation of 4-chloro-7-nitro-1H-indazol-3-amine

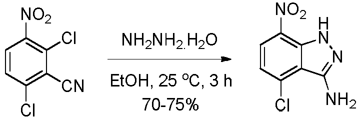

To a stirred solution of 2,6-dichloro-3-nitrobenzonitrile (750.0 g, 3.45 mol, 1.0 equiv.) in ethanol (7.5 L, 10.0 V) at 15-20 °C. was slowly added hydrazine hydrate (519.0 g, 10.36 mol, 3.0 equiv.) while maintaining the reaction mass below 25 °C (Observation: Addition is slightly exothermic and solid formation will begin upon addition). The reaction mixture temperature was slowly raised to room temperature and then the mixture was stirred for 3 h (Observation: the quantity of solids will increase during this time). After completion of the reaction (monitored by TLC), the mixture was diluted with water (7.5 L, 10.0 V) and further stirred for 1 h at room temperature. The solids were isolated via filtration and then were washed with water (2.25 L, 3.0 V). The wet solid was washed with a 1:1 ratio mixture of acetone (1.875 L, 2.5 V) and hexanes (1.875 L, 2.5 V). Bulk residual water was removed from the solids by maintaining vacuum filtration for 60-90 min. The wet solid was finally dried in a hot air oven for 7-8 h at 50 °C (until moisture content reaches below 1.5%) to get the dried product, 4-chloro-7-nitro-1*H-*indazol-3-amine (549.0 g, 75% yield) as a brick red-colored solid. ¹H NMR (400 MHz, CDCl₃): *δ* 10.36 (bs, 1H), 8.20 (d, *J* = 8.4 Hz, 1H), 7.07 (d, *J* = 8.40 Hz, 1H), 4.73 (bs, 2H).

### Step 4: Preparation of 4-chloro-1-methyl-7-nitro-1H-indazol-3-amine

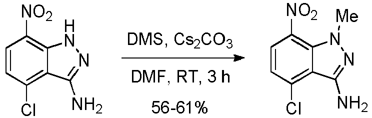

To a stirred solution of 4-chloro-7-nitro-1*H-*indazol-3-amine (500 g, 0.42 mol, 1.0 equiv.) in DMF (5.0 L, 10.0 V) at 5-10 °C was slowly added cesium carbonate (Cs₂CO₃) (1.91 kg, 5.88 mol, 2.5 equiv.) while maintaining the reaction mass below 10 °C. After being stirred for 5-10 min, dimethyl sulphate (326.3 g, 2.59 mol, 1.1 equiv.) was added while maintaining the reaction mass below 10 °C (Note: Slow addition is preferred for obtaining more favorable regio-selectivity). Then, the reaction temperature was slowly raised to room temperature and stirring was continued an additional 2 h at the same temperature. After completion of the reaction (monitored by TLC), the reaction mass was quenched by the addition of ice-cold water (15.0 L, 30.0 V) and the resulting mixture was then stirred for 6-8 h at room temperature. The solids were isolated via filtration and were then washed with water (1.5 L, 3.0 V). The wet solid was washed with IPA (1.5 L, 3.0 V) followed by hexanes (1.0 L, 2.0 V). Bulk residual water was removed from the solids by maintaining vacuum filtration for 60-90 min. The wet solid was dried in a hot air oven for 7-8 h at 50 °C (until moisture content is below 1.0%). The isolated material, 4-chloro-1-methyl-7-nitro-1*H*-indazol-3-amine (319.0 g, 60% yield), was used in the next step without further purification. ¹H NMR (400 MHz, CDCl₃): *δ* 7.97 (d, *J=* 8.32 Hz, 1H), 6.97 (d, *J* = 8.24 Hz, 1H), 4.63 (bs, 2H), 3.96 (s, 3H).

### Step 5: Preparation of N-(4-chloro-1-methyl-7-nitro-1H-indazol-3-yl)methanesulfonamide

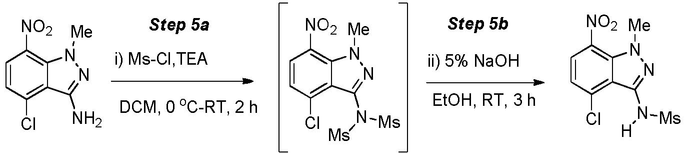

(Step 5a) To a solution of 4-chloro-1-methyl-7-nitro-1*H*-indazol-3-amine (625.0 g, 2.76 mol, 1.0 equiv.) in DCM (6.25 L, 10.0 V) at 0-5 °C. was added triethylamine (TEA) (837.0 g, 8.27 mol, 3.0 equiv.); followed by the addition of 4-dimethylaminopyridine (DMAP) (20.60 g, 0.165 mol, 0.06 equiv.). The reaction mass was stirred for 5-10 min., then methanesulfonyl chloride (MsCl) (790.0 g, 6.89 mol, 2.5 equiv.) added slowly while maintaining the reaction mass below 10 °C. The reaction mixture was allowed to warm to room temperature and was then stirred for 1.5-2.0 h. After completion of the reaction (monitored by TLC), the mixture was diluted with water (6.25 L, 10.0 V) and then stirred at room temperature for 15 min. The organic layer was separated, and the aqueous layer was extracted with DCM (6.25 L, 10.0 V). The combined organic layers were washed with brine (1.25 L, 2.0 V), dried over Na₂SO₄ and concentrated to get the crude solids. The solids were triturated with hexanes (1.25 L, 2.0 V) at room temperature to obtain the intermediate, N-(4-chloro-1-methyl-7-nitro-1H-indazol-3-yl)-N-(methylsulfonyl)methanesulfonamide, which was used directly in the next step. (ii) To a stirred solution of N-(4-chloro-1-methyl-7-nitro-1H-indazol-3-yl)-N-(methylsulfonyl)methanesulfonamide (prepared above) in ethanol (10.5 L, 20.0 V) at room temperature was added slowly an aq. 5% NaOH solution (4.38 L, 7.0 V) [Note: Slow addition is preferred via dropping funnel]. The reaction mass was stirred at the same temperature for 3 h. After completion of the reaction (monitored by TLC) [Sample preparation for TLC analysis: ~1.0 ml of sample acidified with aq. 2.0 N HCl to reach the pH: 2-3, extract it with ethyl acetate and analyze the organic layer by TLC], the reaction mass was cooled to 0-5 °C and the pH was adjusted to 2-3 by the addition of aq. 2.0 N HCl (3.13 L, 5.0 V) while maintain the reaction temperature below 10 °C [Note: Precipitation occurred upon addition of HCl and increased with stirring]. The reaction mixture was warmed to room temperature and then stirred for 1.5-2.0 h. Solids obtained were isolated via filtration and were then washed with water (1.25 L, 2.0 V); followed by washing with hexanes (1.25 L, 2.0 V). Bulk residual water was removed from the solids by maintaining vacuum filtration for 60-90 min. The wet material was dried in a hot air oven at 50 °C for 6-7 h (Until the moisture content is below 1.0%) to get the dried product, *N-*(4-chloro-1-methyl-7-nitro-1*H*-indazol-3-yl)methanesulfonamide (640.0 g, 76%) as a yellow solid. ¹H NMR (400 MHz, CDCl₃): *δ* 8.05 (d, *J=* 8.32 Hz, 1H), 7.32 (bs, 1H), 7.17 (d, *J* = 8.28 Hz, 1H), 4.15 (s, 3H), 3.45 (s, 3H).

### Step 6: Preparation of N-(4-chloro-1-methyl-7-nitro-1H-indazol-3-yl)-N-(4-methoxybenzyl)methanesulfonamide

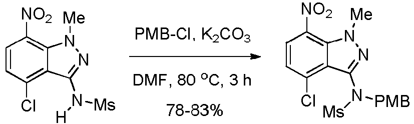

To a mixture of *N*-(4-chloro-1-methyl-7-nitro-1*H*-indazol-3-yl)methanesulfonamide (635.0 g, 2.08 mol, 1.0 equiv.) and 1-(chloromethyl)-4-methoxybenzene (359.0 g, 2.30 mol, 1.1 equiv.) in DMF (6.35 L, 10.0 V) at room temperature was added potassium carbonate (374.7 g, 2.70 mol, 1.3 equiv.). The reaction mixture was heated to 80-90 °C and maintained at that temperature for 3 h. After completion of the reaction (monitored by TLC), the mixture was poured into ice cold water (19.05 L, 30.0 V) [Note: Slow quenching with vigorous stirring is preferred to avoid clumping as the product precipitates]. The resulting solids were isolated via filtration and washed with water (1.90 L, 3.0 V); then the solids were washed with hexanes (1.27 L, 2.0 V). Bulk residual water was removed from the solids by maintaining vacuum filtration for 60-90 min. The isolated solid was dissolved in Ethyl acetate (12.7 L, 20.0 V) and charcoal was added (63.5 g). The mixture was heated to 60-70 °C and then stirred for 30-45 min. at that temperature. The mixture was filtered while still hot (40-50 °C) through a pad of Celite and the Celite pad was then extracted with ethyl acetate (3.17 L, 5.0 V). The combined filtrates were concentrated to dryness under reduced pressure at below 50 °C. Ethyl acetate (0.635 L, 1.0 V) was added to the solids at room temperature. The resultant solid suspension was stirred for 30 min. The solids were isolated via filtration and then were washed with hexanes (1.27 L, 2.0 V). Residual water was removed from the solids by maintaining vacuum filtration for 45-60 min. to afford the product *N*-(4-chloro-1-methyl-7-nitro-1*H*-indazol-3-yl)-*N*(4-methoxybenzyl) methane sulfonamide (705.0 g, 80% yield) as a yellow solid. ¹H NMR (400 MHz, CDCl₃): *δ* 7.99 (d, *J=* 8.24 Hz, 1H), 7.27 (d, *J* = 8.68 Hz, 2H), 7.19 (d, *J=* 8.24 Hz, 1H), 6.80 (d, *J* = 8.44 Hz, 2H), 4.95-4.76 (m, 2H), 4.17 (s, 3H), 3.76 (s, 3H), 3.01 (s, 3H).

### Step 7: Preparation of N-(7-Amino-4-chloro-1-methyl-1H-indazol-3-yl)-N-(4-methoxybenzyl)methanesulfonamide

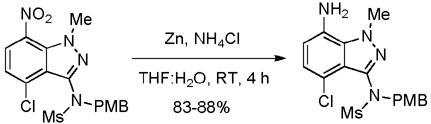

To a stirred suspension of zinc powder (540.0 g, 8.23 mol, 10.0 equiv.) in a mixture of THF (3.50 L, 10.0 V) and water (7.0 L, 20.0 V) at room temperature was added ammonium chloride (NH₄Cl) (449.0 g, 8.23 mol, 10.0 equiv.). To the mixture was added *N-*(4-chloro-1-methyl-7-nitro-1*H*-indazol-3-yl)-*N-*(4-methoxybenzyl)methanesulfonamide (350 g, 0.823 mol, 1.0 equiv.) in THF (7.0 L, 20.0 V). The reaction mixture was stirred at room temperature for 3-4 h. After completion of the reaction (monitored by in-process TLC/HPLC), the mixture was diluted with ethyl acetate (3.5 L, 10.0 V) and water (1.12 L, 2.5 V). The mixture was stirred for 15 min. The reaction mass was filtered through a pad of Celite bed washing with ethyl acetate (1.75 L, 5.0 V). The bi-phasic filtrate was collected, and the phases were separated. The aqueous layer was extracted with ethyl acetate (3.50 L, 10.0 V). The combined organic layers were washed with brine (3.50 L, 10 V), dried over Na₂SO₄, and then concentrated in *vacuo to* afford a crude solid. To the crude product was added MTBE (3.25 L, 10 V) and the suspension was stirred for 30 min at room temperature. The solids were isolated by filtration. Bulk residual water was removed from the solids by maintaining vacuum filtration for 30-45 min. The wet product was dried in a hot air oven (50 °C) for 2 h to afford the title product, *N-*(7-amino-4-chloro-1-methyl-1*H*-indazol-3-yl)-*N*-(4-methoxybenzyl)methanesulfonamide (276.0 g, 85% yield) as off-white solid. ¹H NMR (400 MHz, CDCl₃): *δ*7.29-7.26 (m, 2H), 6.86-6.79 (m, 2H), 6.42 (d, *J=* 7.80 Hz, 1H), 4.99-4.70 (m, 2H), 4.25 (s, 3H), 3.77 (s, 5H), 2.98 (s, 3H).

### Preparation of 2-amino-4-(6-(trifluoromethyl)pyridin-2-yl)benzoic acid:

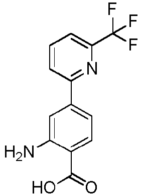

### Synthesis Scheme:

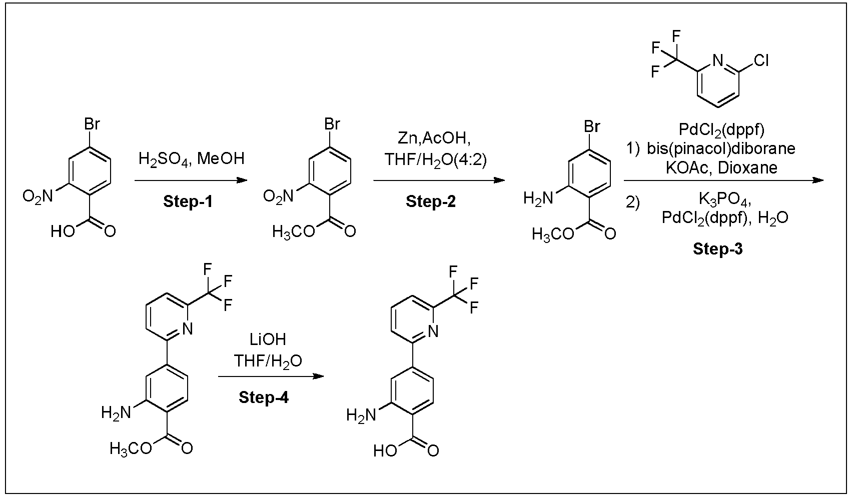

### Step 1: Preparation of methyl 4-bromo-2-nitrobenzoate

To a stirred solution of 4-bromo-2-nitrobenzoic acid (500 g, 2032 mmol) in methanol (2000 mL) was added sulfuric acid (500 mL, 9381 mmol) at 0°C. The solution was stirred for 4 hr at 70 °C. The progress of the reaction was monitored by TLC (SiO₂, 30% EtOAc/Pet. Rf = 0.3). After completion of the reaction, the reaction mass was cooled to room temperature and then concentrated under reduced pressure to remove the methanol. The resulting residue was poured into water (1000 mL) and the pH was adjusted to pH 9 via the addition of anhydrous sodium carbonate. The mixture was extracted with ethyl acetate (2 x 1000 mL). The combined organics were washed with water (500 mL) and then brine solution (500 mL). The organic layer was dried over anhydrous Na₂SO₄, filtered, and then concentrated under reduced pressure to afford methyl 4-bromo-2-nitrobenzoate (520 g, 94%) as an off-white solid. 1H-NMR (400 MHz, CDCl₃) *δ* = 8.04-7.99 (m, 1H), 7.85-7.78 (m, 1H), 7.66 (d, *J* = 8.2 Hz, 1H), 3.92 (s, 3H). LCMS Purity = 95.2%. The product was used directly in the next step without further purification.

### Step 2: Preparation of methyl 2-amino-4-bromobenzoate

To a stirred solution of zinc powder (704 g, 10.8 mol) in water (2000 mL) at 0°C under nitrogen atmosphere was slowly added a solution of methyl 4-bromo-2-nitrobenzoate (400 g, 1538 mmol) in tetrahydrofuran (THF) (4000 mL) followed by acetic acid (1057 mL, 18.5 mol). The reaction mixture was stirred at 27 °C for 4 hr. The progress of the reaction was monitored by TLC (SiO₂, 20% EtOAc/Pet. Rf =0.4). On completion, the reaction mixture was filtered through a Celite pad and the Celite pad was extracted with EtOAc (2000 mL). The combined filtrate was concentrated under reduced pressure. The resulting residue was diluted with water (3000 mL) and extracted with EtOAc (2 x 4000 mL). The combined organics were washed with saturated Na₂CO₃ solution (2 x 3000 mL) and then brine (2 x 2000 mL). The organic layer was dried over anhydrous Na₂SO₄, filtered, and then concentrated under reduced pressure to afford methyl 2-amino-4-bromobenzoate (350 g, 94%) as an off white solid. 1H-NMR (400 MHz, CDCl₃) *δ* = 7.76-7.65 (m, 1H), 6.84 (d, *J=* 1.9 Hz, 1H), 6.78-6.72 (m, 1H), 5.91-5.63 (m, 2H), 3.86 (s, 3H). LCMS Purity = 95.0%. The product was used directly in the next step without further purification.

### Step 3: Preparation of methyl 2-amino-4-(6-(trifluoromethyl)pyridin-2-yl)benzoate

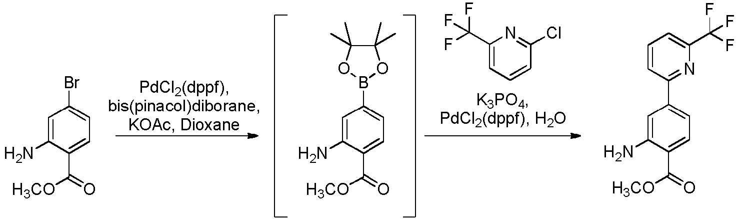

To a stirred solution of methyl 2-amino-4-bromobenzoate (350 g, 1521 mmol) in 1,4-dioxane (7000 mL) was added bis(pinacol)diborane (522 g, 2054 mmol) and potassium acetate (597 g, 6085 mmol). The reaction mixture was degassed by bubbling N₂ gas through the mixture for 10 min. To the reaction mixture was added PdCl₂(dppf) (78 g, 106 mmol). The mixture was stirred at 90 °C for 4 hr. The progress of the reaction was monitored by TLC. On completion of the reaction the mixture was cooled to room temperature. To the mixture was added 2-chloro-6-(trifluoromethyl)pyridine (359 g, 1978 mmol), tribasic potassium phosphate (1130 g, 5325 mmol) and water (1190 mL). The mixture was degassed via nitrogen gas bubbling for 10 mins. To the mixture was added PdCl₂(dppf) (78 g, 106 mmol). The reaction mixture was stirred at 60 °C for 16 hr. The progress of the reaction was monitored by TLC (SiO₂, 20% EtOAc/Pet. Rf = 0.4). On completion, the reaction mixture was filtered through Celite and the Celite pad was then extracted with ethyl acetate (2000 mL). The combined filtrate was concentrated under reduced pressure to afford crude the crude product (550 g) as a brown liquid. This material was purified via silica gel chromatography eluting with 5-30% EtOAc/Pet. The fractions containing the desired product were pooled and concentrated under reduced pressure. The isolated material was washed with n-pentane (2200 mL) and the solids were collected via filtration and then dried under vacuum to afford methyl 2-amino-4-(6-(trifluoromethyl)pyridin-2-yl)benzoate (380 g, 83%) as an off white solid. ¹H NMR (CHLOROFORM-d) δ: 7.99 (d, J=8.3 Hz, 1H), 7.92-7.98 (m, 2H), 7.67 (dd, J=6.9, 1.5 Hz, 1H), 7.49 (d, J=1.5 Hz, 1H), 7.27 (dd, J=8.3, 1.8 Hz, 1H), 5.90 (br s, 2H), 3.93 (s, 3H). LCMS Purity = 98.25%.

### Step 4: Preparation of 2-amino-4-(6-(trifluoromethyl)pyridin-2-yl)benzoic acid:

To a stirred solution of methyl 2-amino-4-(6-(trifluoromethyl)pyridin-2-yl)benzoate (650 g, 2194 mmol) in tetrahydrofuran (THF) (5000 mL) and water (2167 mL) at 0 °C under nitrogen atmosphere was added lithium hydroxide monohydrate (369 g, 8776 mmol). The reaction mixture was stirred at 70°C for 16 hr. The progress of the reaction was monitored by TLC (SiO₂, 50% EtOAc/Pet. Rf = 0.4). On completion, the reaction mixture was concentrated under reduced pressure and the resulting residue was then dissolved in water (5000 mL) and acidified to pH 4 via the addition of 3N HCl (3000 mL). The resulting precipitate was collected via filtration and was washed with water (4000 mL), then n-hexane (5000 mL), and then dried to afford 2-amino-4-(6-(trifluoromethyl)pyridin-2-yl)benzoic acid (581 g, 93%) as an off-white solid. 1H-NMR (400 MHz, DMSO-d₆) *δ* = 8.18 (d, *J* = 4.0 Hz, 2H), 7.92-7.88 (m, 1H), 7.84 (d, *J=* 8.3 Hz, 1H), 7.54 (d, *J=* 1.7 Hz, 1H), 7.22 (d, *J* = 8.4, 1.8 Hz, 1H). LCMS Purity = 99.62%.

### Preparation of Example 1: N-((S)-1-((3P)-3-(4-chloro-1-methyl-3-(methylsulfonamido)-1H-indazol-7-yl)-4-oxo-7-(6-(trifluoromethyl)pyridin-2-yl)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide.

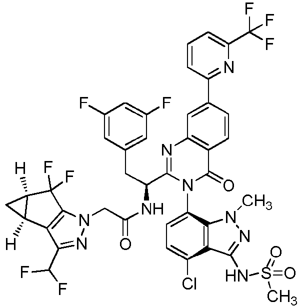

### Synthesis Scheme:

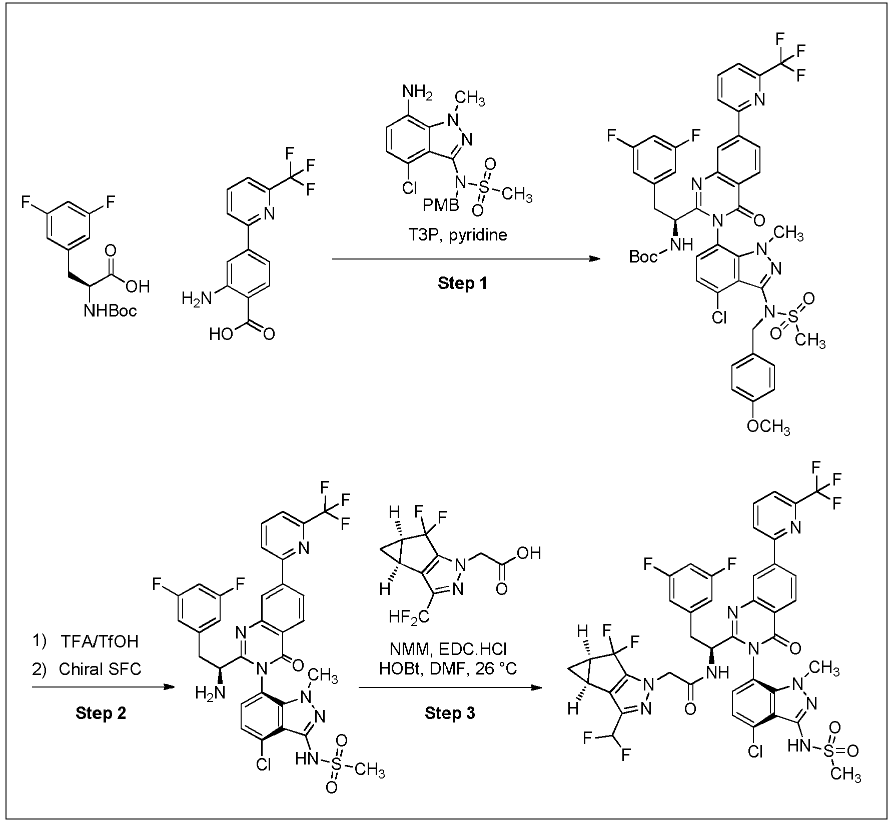

### Step 1: Preparation of tert-butyl (S)-(1-(3-(4-chloro-3-(N-(4-methoxybenzyl)methylsulfonamido)-1-methyl-1H-indazol-7-yl)-4-oxo-7-(6-(trifluoromethyl)pyridin-2-yl)-3,4-dihydroquinazolin-2-yl)-2-(3,5-difluorophenyl)ethyl)carbamate

To a stirred solution of (S)-2-((tert-butoxycarbonyl)amino)-3-(3,5-difluorophenyl) propanoic acid (91 g, 301 mmol) and 2-amino-4-(6-(trifluoromethyl)pyridin-2-yl)benzoic acid (94 g, 332 mmol) in acetonitrile (3.8 L)) under nitrogen atmosphere at 27 °C was added pyridine (0.059 L, 724 mmol). The resulting mixture was cooled to -9 °C for 10 min., then 2,4,6-tripropyl-1,3,5,2,4,6-trioxatriphosphinane 2,4,6-trioxide ("T3P", 50% wt in EtOAc, 0.888 L, 1507 mmol) was added drop-wise over 10 min. The solution was stirred for 2.1 hr at -9°C under N₂ atmosphere. To the solution at -9°C was added N-(7-amino-4-chloro-1-methyl-1H-indazol-3-yl)-N-(4-methoxybenzyl)methanesulfonamide (120 g, 301 mmol) upon which the solution warmed to -5 °C and was maintained at that temperature with stirring for 1 hr. The reaction mass was then allowed to slowly warm to 27 °C and was then stirred at that temperature for 16 hrs. The progress of the reaction was monitored by TLC (SiO₂, 50% EtOAc/Pet. Rf = 0.5). On completion, the reaction mixture was concentrated under reduced pressure and the resulting residue was dissolved in EtOAc (5000 mL) and then washed with 1N NaOH solution (2000 mL) followed by brine (1000 mL). The organic layer was dried over Na₂SO₄, filtered, and then concentrated under reduced pressure to afford the crude product which was purified by silica gel chromatography eluting with 30-35% EtOAc/Pet. The fractions containing the desired product were pooled and concentrated under reduced pressure to afford tert-butyl (S)-(1-(3-(4-chloro-3-(N-(4-methoxybenzyl)methylsulfonamido)-1-methyl-1H-indazol-7-yl)-4-oxo-7-(6-(trifluoromethyl)pyridin-2-yl)-3,4-dihydroquinazolin-2-yl)-2-(3,5-difluorophenyl)ethyl)carbamate (252 g, 88%, an off-white solid) as a mixture of homochiral atropisomers (diastereomers).

### Step 2: Preparation of (S)-N-((6P)-7-(2-(1-amino-2-(3,5-difluorophenyl)ethyl)-4-oxo-7-(6-(trifluoromethyl)pyridin-2-yl)quinazolin-3(4H)-yl)-4-chloro-1-methyl-1H-indazol-3-yl)methanesulfonamide

To a stirred solution of tert-butyl (S)-(1-(3-(4-chloro-3-(N-(4-methoxybenzyl)methylsulfonamido)-1-methyl-1H-indazol-7-yl)-4-oxo-7-(6-(trifluoromethyl)pyridin-2-yl)-3,4-dihydroquinazolin-2-yl)-2-(3,5-difluorophenyl)ethyl)carbamate (97% purity, 252 g, 264 mmol) in TFA (815 ml, 10.6 mol) at 27 °C was added triflic acid (70.4 ml, 793 mmol). The solution was stirred for 2h under nitrogen atmosphere. The progress of the reaction was monitored by TLC (SiO₂, 50% EtOAc/Pet. Rf = 0.2). On completion, the volatiles were removed under a gentle stream of nitrogen gas. The residue was dissolved in EtOAc (5000 mL) and then washed with 1N NaOH solution (2000 mL) followed by brine (1500 mL). The organic layer was dried over Na₂SO₄, filtered, and then concentrated under reduced pressure to afford the crude product which was purified by silica gel chromatography eluting with 5-15% MeOH in DCM. The fractions containing the desired product were pooled and concentrated under reduced pressure to afford (S)-N-(7-(2-(1-amino-2-(3,5-difluorophenyl)ethyl)-4-oxo-7-(6-(trifluoromethyl)pyridin-2-yl)quinazolin-3(4H)-yl)-4-chloro-1-methyl-1H-indazol-3-yl) methane sulfonamide (180 g, 95%, an off-white solid) as a mixture of homochiral atropisomers (diastereomers). The material was dissolved in methanol:acetonitrile (40:60, 3000 mL) and was then purified by prep-SFC using the following method: Column = (R,R) Welk-01, 30x250 mm, 5µ; eluent = CO₂:methanol (1:1); Flow-rate = 90.0 g/min.; Back-pressure = 120.0 bar; Detection = 254 nm (UV); Stack time = 8.8 min.; Load per injection = 700 mg. The SFC separation produced two peaks which were collected separately. The major peak (second peak to elute) was concentrated under reduced pressure to afford (S)-N-((6P)-7-(2-(1-amino-2-(3,5-difluorophenyl)ethyl)-4-oxo-7-(6-(trifluoromethyl)pyridin-2-yl)quinazolin-3(4H)-yl)-4-chloro-1-methyl-1H-indazol-3-yl)methanesulfonamide (100 g, 54%) as an off-white solid. The product is a single stereoisomer. 1H-NMR (400 MHz, DMSO-d₆) *δ* = 8.64-8.55 (m, 2H), 8.44-8.25 (m, 3H), 8.01 (d, *J=* 7.7 Hz, 1H), 7.42-7.31 (m, 2H), 7.07-6.95 (m, 1H), 6.76 (dd, *J* = 2.0, 8.5 Hz, 2H), 3.70 (s, 3H), 3.59 (dd, *J* = 4.8, 8.2 Hz, 1H), 3.35 (br d, *J =* 4.8 Hz, 1H), 3.17 (d, *J=* 5.1 Hz, 3H), 2.92-2.83 (m, 1H). LCMS Purity = 99%.

### Step 3: Preparation of N-((S)-1-((3P)-3-(4-chloro-1-methyl-3-(methylsulfonamido)-1H-indazol-7-yl)-4-oxo-7-(6-(trifluoromethyl)pyridin-2-yl)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide:

To a stirred solution of (S)-N-((6P)-7-(2-(1-amino-2-(3,5-difluorophenyl)ethyl)-4-oxo-7-(6-(trifluoromethyl)pyridin-2-yl)quinazolin-3(4H)-yl)-4-chloro-1-methyl-1H-indazol-3-yl)methanesulfonamide (45 g, 63.9 mmol) in DMF (450 mL) at 27 °C was added 2-((3bS,4aR)-3-(difluoromethyl)-5,5-difluoro-3b,4,4a,5-tetrahydro-1H-cyclopropa [3,4] cyclopenta[1,2-c]pyrazol-1-yl)acetic acid (20.26 g, 77 mmol) followed by N-(3-dimethylaminopropyl)-N'-ethylcarbodiimide hydrochloride ("EDC-HCl", 14.70 g, 77 mmol), 1-hydroxybenzotriazole hydrate ("HOBt hydrate", 11.75 g, 77 mmol) and N-methylmorpholine (28.1 mL, 256 mmol). The reaction mass was stirred for 24 hr at 27 °C. The progress of the reaction was monitored by TLC (SiO₂, 50% EtOAc/Pet. Rf = 0.5). On completion, the reaction mass was diluted with ice water (1.5 L) and the resulting precipitate was collected via filtration and then dried under vacuum to afford the crude product (59 g) as an off-white solid. This crude product was blended with another batch of crude product (61 g) generated by repeating the procedure on the same scale. Together, the 120 g of crude product was purified by silica gel chromatography eluting with 20-40% EtOAc/Pet. The fractions containing the desired product were pooled and concentrated under reduced pressure to afford the purified product. Trace EtOAc residue was removed by grinding the compound using a mortar and pestle and then maintaining the fine solids in a 50°C oven for approximately 2h; this process of grinding and heating was repeated an additional 4 times until the EtOAc content was reduced to below 4000 ppm to afford N-((S)-1-((3P)-3-(4-chloro-1-methyl-3-(methylsulfonamido)-1H-indazol-7-yl)-4-oxo-7-(6-(trifluoromethyl)pyridin-2-yl)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (85.7 g, 79%) as an off-white solid. ¹H NMR (acetone-d6) δ: 8.60 (t, J=1.0 Hz, 1H), 8.58 (s, 1H), 8.48 (d, J=8.0 Hz, 1H), 8.39 (d, J=0.9 Hz, 2H), 8.32 (t, J=7.9 Hz, 1H), 8.16 (d, J=8.6 Hz, 1H), 7.95-7.99 (m, 1H), 7.49 (d, J=7.7 Hz, 1H), 7.37 (d, J=8.0 Hz, 1H), 6.86 (tt, J=9.3, 2.3 Hz, 1H), 6.70-6.76 (m, 2H), 6.77 (t, J=54.7 Hz, 1H), 4.93 (td, J=9.0, 4.6 Hz, 1H), 4.63-4.74 (m, 2H), 3.69 (s, 3H), 3.55 (dd, J=14.2, 4.6 Hz, 1H), 3.28 (s, 3H), 3.15 (dd, J=14.2, 9.4 Hz, 1H), 2.42-2.51 (m, 2H), 1.37-1.43 (m, 1H), 0.95-1.00 (m, 1H). LCMS analysis method: Column: Acquity BEH C18, 2.1 x 50 mm, 1.7 µm particles; Solvent A = 0.1% Formic acid in Water; Solvent B = 0.1% Formic Acid in Acetonitrile; Flow Rate = 0.6 mL/min.; Gradient {time-point (min.) / %B at time-point (%)} = 0/3, 0.4/3, 7.5/98, 9.5/98, 9.6/3, 10/3; Column Temperature = 35 °C. LCMS result: retention time = 5.57 mins.; observed ion = 949.98 (M+H); LCMS Purity = 99.4%.

### Naming of Example 1:

The compound of Example 1 as prepared above is a homochiral material that contains axial chirality. Axial chirality can be described using P/M nomenclature as detailed in the IUPAC Gold Book (doi:10.1351/goldbook.A00547). However, at this time only a limited number of software tools are available which are capable of generating chemical names containing P/M nomenclature, and even fewer options are available to convert chemical names using this nomenclature to structural representations of the molecules. Therefore, for clarity and convenience several names for Example 1 are provided below:
The name of Example 1 as generated by ChemDraw Professional 16 (absent P/M nomenclature) is:
   N-((S)-1-(3-(4-chloro-1-methyl-3-(methylsulfonamido)-1H-indazol-7-yl)-4-oxo-7-(6-(trifluoromethyl)pyridin-2-yl)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide
The chemical name of Example 1 as generated by JChem for Excel (including P/M nomenclature) is:
   N-[(1S)-1-[3-(4-chloro-3-methanesulfonamido-1-methyl-1H-indazol-7-yl)-4-oxo-7-[6-(trifluoromethyl)pyridin-2-yl]-3,4-dihydroquinazolin-2-yl]-2-(3,5-difluorophenyl)ethyl]-2-[(2S,4R)-9-(difluoromethyl)-5,5-difluoro-7,8-diazatricyclo[4.3.0.0²,⁴]nona-1(6),8-dien-7-yl]acetamide
The chemical name of Example 1 generated by ChemDraw Professional 16 with manually added P/M nomenclature is:
   N-((S)-1-((3P)-3-(4-chloro-1-methyl-3-(methylsulfonamido)-1H-indazol-7-yl)-4-oxo-7-(6-(trifluoromethyl)pyridin-2-yl)-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

### Preparation of 2-amino-4-(4-(difluoromethyl)pyrimidin-2-yl)benzoic acid:

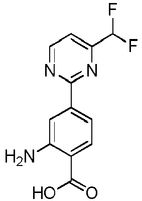

### Synthesis Scheme:

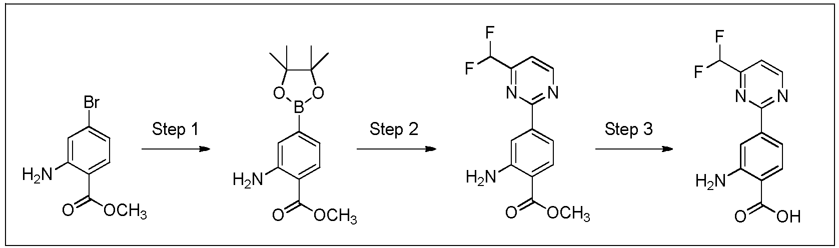

### Step 1: Preparation of methyl 2-amino-4-(4,4,5,5-tetramethyl-1,3,2-dioxaborolan-2-yl)benzoate

A mixture of methyl 2-amino-4-bromobenzoate (10 g, 43.5 mmol), 4,4,4',4',5,5,5',5'-octamethyl-2,2'-bi(1,3,2-dioxaborolane) (11.04 g, 43.5 mmol), PdCl₂(dppf) (1.590 g, 2.173 mmol), and potassium acetate (12.80 g, 130 mmol) in 1,4-dioxane (100 mL) under argon was heated at 97 °C for 2h. The mixture was cooled to room temperature and then was diluted with DCM. The organic layer was washed with water, followed by brine, dried over MgSO₄, filtered, and concentrated under reduced pressure to afford the crude product. The crude product was purified via silica gel chromatography (330 g column, 5-30% EtOAc:Hex) to afford the product methyl 2-amino-4-(4,4,5,5-tetramethyl-1,3,2-dioxaborolan-2-yl)benzoate (9.2 g, 76 %) as a yellow solid. ¹H NMR (500 MHz, CDCl₃) δ ppm 7.86 (d, *J*=8.05 Hz, 1 H), 7.14 (s, 1 H), 7.06 (dd, *J*=7.90, 1.04 Hz, 1 H), 5.67 (br s, 2 H), 3.89 (s, 3 H), 1.37 (s, 12 H).

### Step 2: Preparation of methyl 2-amino-4-(4-(difluoromethyl)pyrimidin-2-yl)benzoate

In a round-bottom flask was combined methyl 2-amino-4-(4,4,5,5-tetramethyl-1,3,2-dioxaborolan-2-yl)benzoate (14.8 g, 53.4 mmol), 2-chloro-4-(difluoromethyl)pyrimidine (8.79 g, 53.4 mmol), PdCl₂(xantphos) (2.019 g, 2.67 mmol) and potassium carbonate (22.14 g, 160 mmol). The flask was sealed with a rubber septum and to the flask was added 1,4-dioxane (200 mL) and water (50.0 mL). The flask was then backfilled with argon (vac then backfill with argon 3 times). The mixture was stirred at 60 °C for 3.5 h. The mixture was cooled to room temperature and the volatile organics were removed under reduced pressure to afford an aqueous mixture. The slurry was taken up in EtOAc (300 mL) and then was further diluted with water (400 mL). The mixture was mixed and then filtered through a Celite pad to removed insoluble material. The organic layer was then separated and washed with brine, dried over MgSO₄, filtered and concentrated under reduced pressure. The residue was taken up in a minimum amount of EtOAc (100 mL) and was then mixed with Celite and concentrated under reduced pressure to afford a free-flowing powder. This powder was separated into three equal portions and each portion was subjected to reverse phase chromatography (415g RediSep Gold C18 column) eluting with (95:5 water:MeCN + 0.1% formic acid):(95:5 MeCN:water + 0.1% Formic acid) 25:75→0:100. Fractions containing the desired product were pooled and partially concentrated under reduced pressure to afford an aqueous mixture. The slurry was combined with EtOAc and the aqueous layer was made slightly basic (pH 8) by the addition of aq. 5 N NaOH. The mixture was mixed and then the organic layer was isolated and washed with brine, dried over MgSO₄, and concentrated under reduced pressure to afford the product methyl 2-amino-4-(4-(difluoromethyl)pyrimidin-2-yl)benzoate (10.2 g, 68%) as a dark yellow solid. ¹H NMR (500 MHz, CDCl₃) δ ppm 9.02 (br d, *J*=4.77 Hz, 1 H), 8.01 (d, *J*=8.34 Hz, 1H), 7.86 (s, 1 H), 7.76 (br d, *J*=8.35 Hz, 1 H), 7.55 (br d, *J*=4.77 Hz, 1 H), 6.52 - 6.78 (m, 1 H), 5.88 (br s, 2 H), 3.94 (s, 3 H). LCMS Method G: retention time = 2.66 mins.; observed ion = 321.1 (M+MeCN).

### Step 3: Preparation of 2-amino-4-(4-(difluoromethyl)pyrimidin-2-yl)benzoic acid

To a solution of methyl 2-amino-4-(4-(difluoromethyl)pyrimidin-2-yl)benzoate (12 g, 43.0 mmol) in methanol (50 mL) and THF (50.0 mL) was added aqueous 5 N sodium hydroxide (25.8 mL, 129 mmol) and the mixture was then stirred at 60 °C for 1 h upon which LCMS analysis indicated the reaction was complete. The mixture was cooled to room temperature and then to the mixture was added aqueous 1 M HCl (129 mL, 129 mmol). To the thick yellow slurry was added EtOAc (250 mL) and water (150 mL) upon which the yellow slurry partially dissolved; the organic layer was cloudy while the aqueous layer appeared homogeneous. The organic layer was washed with brine and remained cloudy. The organic layer was isolated and heated until the cloudy mixture became a clear yellow solution. The solution was dried over MgSO₄, filtered and concentrated under reduced pressure to afford the product 2-amino-4-(4-(difluoromethyl)pyrimidin-2-yl)benzoic acid (11.3 g, 99 %) as a yellow solid. ¹H NMR (500 MHz, CD₃OD) δ ppm 9.06 (d, *J*=5.07 Hz, 1 H), 7.96 (d, *J*=8.35 Hz, 1 H), 7.93 (d, *J*=1.19 Hz, 1 H), 7.67 (dd, *J*=8.49, 1.64 Hz, 1 H), 7.64 (d, *J*=5.07 Hz, 1 H), 6.66 - 6.91 (m, 1 H). LCMS Method G: retention time = 2.14 mins.; observed ion = 307.0 (M+MeCN).

### Alternate preparation of 2-amino-4-(4-(difluoromethyl)pyrimidin-2-yl)benzoic acid:

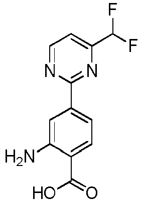

### Synthesis Scheme:

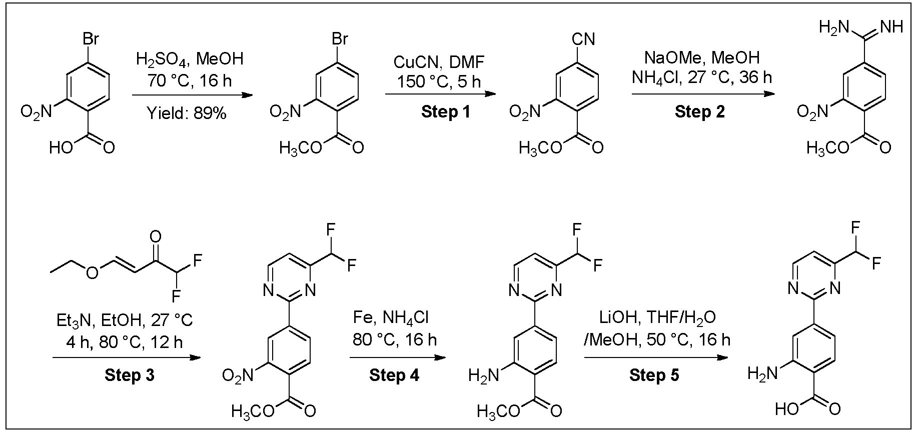

### Preparation of methyl 4-bromo-2-nitrobenzoate:

This compound was prepared on 200 g scale by following the reported procedure of WO 2005037796 and J. Am. Chem. Soc., 2018, 140 (33), 10553-10561.

### Step 1: Preparation of methyl 4-cyano-2-nitrobenzoate

To a stirred solution of methyl 4-bromo-2-nitrobenzoate (340 g, 1307 mmol) in DMF (3000 mL) under nitrogen at 27 °C was added copper (I) cyanide (234 g, 2615 mmol). The reaction mixture was then stirred at 150 °C for 5 h. Progress of the reaction was monitored by TLC (SiO₂, 20% EtOAc/Pet., Rf = 0.6, UV-active). On completion, the reaction mixture was allowed to cool to 27 °C. The reaction mixture was poured into EtOAc (5000 mL) and the resulting mixture was washed with aq. 5% ethylenediamine (5000 mL) to remove copper salts. The organic solution was then washed with ice cold water (3 × 3000 mL) followed by ice cold brine (3000 mL). The organic layer was dried over Na₂SO₄, filtered, and concentrated under reduced pressure to afford methyl 4-cyano-2-nitrobenzoate as a brown solid, 280 g (83%). The product was used directly in the next step without further purification. ¹HNMR (400 MHz, CDCl₃) *δ* = 8.37 (d, *J* = 1.6 Hz, 1H), 7.97 (dd, *J* = 7.9, 1.6 Hz, 1H), 7.87 (d, *J* = 7.9 Hz, 1H), 3.97 (s, 3H). Note: The work-up process was further optimized to avoid the need for filtration: The reaction mixture was poured into EtOAc (5000 mL) and the resulting mixture was washed with aq. 5% ethylenediamine (5000 mL) to remove copper salts. The organic solution was then washed with ice cold water (3 × 3000 mL) followed by ice cold brine (3000 mL). The remaining process is the same as described above.

### Step 2: Preparation of methyl 4-carbamimidoyl-2-nitrobenzoate hydrochloride

To a stirred solution of methyl 4-cyano-2-nitrobenzoate (280 g, 1358 mmol) in MeOH (4000 mL) under nitrogen was added sodium methoxide (44.0 g, 815 mmol) and the reaction mixture was then stirred at 27 °C for 16 h. To the reaction mixture was added ammonium chloride (72.6 g, 1358 mmol) and the reaction mixture was then stirred at 27 °C for 18 h. Progress of the reaction was monitored by TLC (SiO₂, 80% EtOAc/Pet., Rf = 0.1, UV-active). On completion the reaction mixture was filtered, and the filter cake was extracted with 10% MeOH in DCM (3 × 1000 mL). The combined filtrate was concentrated under reduced pressure to obtain the crude product as a gummy solid. This material was triturated with EtOAc (1000 mL) to afford methyl 4-carbamimidoyl-2-nitrobenzoate hydrochloride as a yellow solid, 250 g (56%). The product was used directly in the next step without further purification. ¹H-NMR (400 MHz, DMSO-d₆) *δ* = 9.01 (br s, 3H), 8.50 (d, *J=* 1.6 Hz, 1H), 8.23 (dd, *J=* 7.9, 1.6 Hz, 1H), 8.08 (d, *J=* 7.9 Hz, 1H), 3.91 (s, 3H). LCMS purity = 79%.

### Preparation of (E)-4-ethoxy-1,1-difluorobut-3-en-2-one:

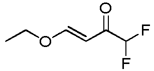

To a stirred solution of 2,2-difluoroacetic anhydride (179 mL, 1436 mmol) in DCM (1250 mL) at 0 °C was added dropwise over 1h a mixture of pyridine (128 mL, 1580 mmol) and ethoxyethene (165 mL, 1724 mmol). The reaction mixture was allowed to warm to 27 °C and was then stirred for 12 h. The reaction mixture was quenched by the addition of ice-cold water (1000 mL). The organic layer was separated and washed with aq. sat. NaHCO₃ (1000 mL), then brine (1000 mL), dried over Na₂SO₄, and filtered. The filtrate was carefully concentrated under reduced pressure (pressure ≥ 100 mbar; bath temperature ≤ 25 °C) to afford (*E*)-4-ethoxy-1,1-difluorobut-3-en-2-one as a brown liquid, 180 g (80%). The crude compound was used directly in the next step without further purification. ¹HNMR (400 MHz, CDCl₃) *δ* = 7.84 (d, *J* = 12.4 Hz, 1H), 5.89-5.63 (m, 2H), 4.06 (q, *J* = 7.0 Hz, 2H), 1.39 (t, J = 7.0 Hz, 3H). GC-MS purity = 95%.

### Step 3: Preparation of methyl 4-(4-(difluoromethyl)pyrimidin-2-yl)-2-nitrobenzoate

To a stirred solution of methyl 4-carbamimidoyl-2-nitrobenzoate (200 g, 708 mmol) in EtOH (2000 mL) in a 5L autoclave flask under nitrogen atmosphere at 27 °C was added (*E*)-4-ethoxy-1,1-difluorobut-3-en-2-one (159 g, 1062 mmol) followed by triethylamine (296 mL, 2124 mmol). The reaction mixture was stirred at 80 °C for 16 h. Progress of the reaction was monitored by TLC (SiO₂, 20% EtOAc/Pet., Rf = 0.5, UV-active). The reaction mixture was filtered and the filtrate was concentrated under reduced pressure to afford methyl 4-(4-(difluoromethyl)pyrimidin-2-yl)-2-nitrobenzoate as a brown liquid, 220 g (58%). The product was used directly the next step without further purification. ¹H-NMR (400 MHz, CDCl₃) *δ* = 9.07-9.00 (m, 1H), 8.80 (dd, *J* = 7.9, 1.6 Hz, 1H), 7.88 (d, *J* = 7.9 Hz, 1H), 7.78-7.56 (m, 1H), 7.62 (d, *J* = 4.8 Hz, 1H), 6.65 (t, *J* = 54.8 Hz, 1H), 3.96 (s, 3H). HPLC Purity: 58%.

### Step 4: Preparation of methyl 2-amino-4-(4-(difluoromethyl)pyrimidin-2-yl)benzoate

To a stirred solution of methyl 4-(4-(difluoromethyl)pyrimidin-2-yl)-2-nitrobenzoate (220 g, 711 mmol) in EtOH (2150 mL) and water (215 mL) at 27 °C was added ammonium chloride (190 g, 3557 mmol) followed by iron (199 g, 3557 mmol). The reaction mixture was stirred at 80 °C for 16 h. Progress of the reaction was monitored by TLC (SiO₂, 20% EtOAc/Pet., Rf = 0.4, UV-active). On completion, the reaction mixture was filtered while hot through a Celite pad and the Celite pad was then extracted with EtOAc (4 × 500 mL). The combined filtrate was concentrated under reduced pressure to afford methyl 2-amino-4-(4-(difluoromethyl)pyrimidin-2-yl)benzoate as a yellow solid, 230 g (64%). The product was used directly in the next step without any further purification. ¹H-NMR (400 MHz, CDCl₃) *δ* = 8.99 (d, *J* = 4.8 Hz, 1H), 7.98 (d, *J=* 8.3 Hz, 1H), 7.83 (s, 1H), 7.73 (d, *J=* 8.4 Hz, 1H), 7.52 (d, *J* = 4.8 Hz, 1H), 6.61 (t, *J* = 54.8 Hz, 1H), 3.91 (s, 3H). LCMS purity = 56%.

### Step 5: Preparation of 2-amino-4-(4-(difluoromethyl)pyrimidin-2-yl)benzoic acid

To a stirred solution of methyl 2-amino-4-(4-(difluoromethyl)pyrimidin-2-yl)benzoate (230 g, 461 mmol) in THF (2300 mL), MeOH (575 mL) and water (192 mL) at 27 °C was added LiOH (66.3 g, 2767 mmol). The reaction mixture was stirred at 50 °C for 16 h. Progress of the reaction was monitored by TLC (SiO₂, 40% EtOAc/Pet., Rf = 0.1, UV-active). On completion the reaction mixture was allowed to cool to 27 °C and was then concentrated under reduced pressure. The crude residue was dissolved in water (1000 mL) and washed with EtOAc (2 × 250 mL). The aqueous layer was acidified with aq. 1N HCl to pH ~6. The precipitated solid was collected via filtration and wash with water (500 mL), then n-pentane (500 mL) and then dried to afford 2-amino-4-(4-(difluoromethyl)pyrimidin-2-yl)benzoic acid as a yellow solid, 80 g (63%). ¹H-NMR (400 MHz, DMSO-d₆) *δ* = 9.15 (d, *J* = 4.8 Hz, 1H), 7.87-7.84 (m, 2H), 7.74 (d, *J=* 4.8 Hz, 1H), 7.53 (d, *J=* 8.4 Hz, 1H), 7.06 (t, *J=* 54.2 Hz, 1H). LCMS purity = 96%.

### Preparation of Example 2: N-((S)-1-((3P)-3-(4-chloro-1-methyl-3-(methylsulfonamido)-1H-indazol-7-yl)-7-(4-(difluoromethyl)pyrimidin-2-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

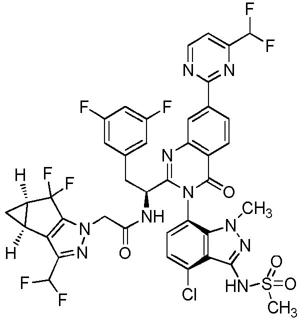

### Synthesis Scheme:

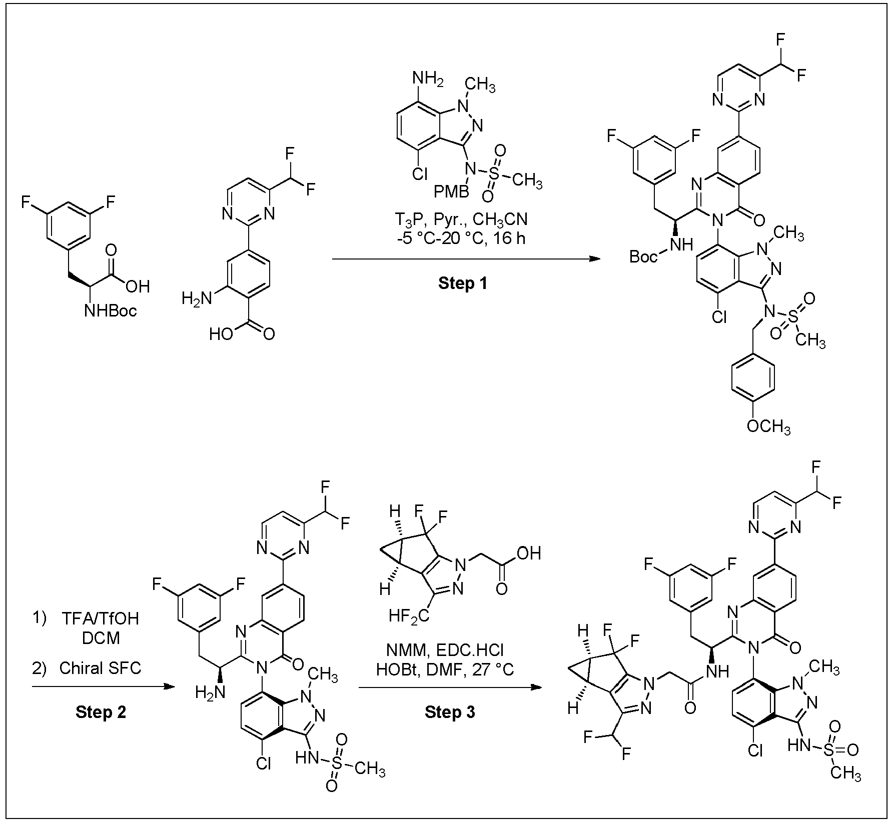

### Step 1: Preparation of tert-butyl (S)-(1-(3-(4-chloro-3-(N-(4-methoxybenzyl)methylsulfonamido)-1-methyl-1H-indazol-7-yl)-7-(4-(difluoromethyl)pyrimidin-2-yl)-4-oxo-3,4-dihydroquinazolin-2-yl)-2-(3,5-difluorophenyl)ethyl)carbamate

To a stirred solution of (S)-2-((tert-butoxycarbonyl)amino)-3-(3,5-difluorophenyl)propanoic acid (76 g, 253 mmol) and 2-amino-4-(4-(difluoromethyl)pyrimidin-2-yl)benzoic acid (73.9 g, 279 mmol) in acetonitrile (2.1 L) was added pyridine (0.049 L, 608 mmol) and the reaction mixture was cooled to -5 °C and stirred at same temperature for 10 min. Then to the reaction mixture at -5 °C was slowly added T3P (50% in EtOAc) (0.754 L, 1266 mmol). The mixture was stirred at -5 °C for 20 min., then was allowed to warm to 27 °C, and then was stirred for 2 h. To the reaction mixture was added N-(7-amino-4-chloro-1-methyl-1H-indazol-3-yl)-N-(4-methoxybenzyl) methanesulfonamide (100 g, 253 mmol) in one portion at 27 °C and the mixture was then stirred for 18 h. The progress of the reaction was monitored by TLC (SiO₂, 40% EtOAc/Pet., Rf = 0.4, UV-active). The reaction mixture was concentrated under reduced pressure to remove acetonitrile and then was diluted with EtOAc (1000 mL) and washed with water (2000 mL). The organic layer was separated and washed with sat. aq. Na₂CO₃ (3 × 500 mL) and then brine (500 mL), dried over anhydrous Na₂SO₄, filtered and concentrated under reduced pressure to afford the crude product as a brown gummy liquid which was purified by column chromatography on silica gel eluting with 30-40% EtOAc/Pet. The fractions containing the desired product were collected and concentrated under reduced pressure to afford tert-butyl (S)-(1-(3-(4-chloro-3-(N-(4-methoxybenzyl)methylsulfonamido)-1-methyl-1H-indazol-7-yl)-7-(4-(difluoromethyl)pyrimidin-2-yl)-4-oxo-3,4-dihydroquinazolin-2-yl)-2-(3,5-difluorophenyl)ethyl)carbamate (180 g, 78 %, a yellow solid) as a mixture of homochiral atropisomers (diastereomers).

### Step 2: Preparation of (S)-N-((6P)-7-(2-(1-amino-2-(3,5-difluorophenyl)ethyl)-7-(4-(difluoromethyl)pyrimidin-2-yl)-4-oxoquinazolin-3(4H)-yl)-4-chloro-1-methyl-1H-indazol-3-yl)methanesulfonamide

To a stirred solution of tert-butyl (S)-(1-(3-(4-chloro-3-(N-(4-methoxybenzyl)methylsulfonamido)-1-methyl-1H-indazol-7-yl)-7-(4-(difluoromethyl)pyrimidin-2-yl)-4-oxo-3,4-dihydroquinazolin-2-yl)-2-(3,5-difluorophenyl)ethyl)carbamate (110 g, 121 mmol) in DCM (500 mL) at 27 °C under N₂ atmosphere was added TFA (374 mL, 4849 mmol) and the solution was stirred for 10 min. To the solution was added trifluoromethanesulfonic acid (32.3 mL, 364 mmol) and the solution was stirred for 1 h at 27 °C. The progress of the reaction was monitored by TLC (SiO₂, 50% EtOAc/Pet., Rf = 0.2). The volatiles were removed under a gentle stream of nitrogen gas. The resulting residue was dissolved in EtOAc (1500 mL) and then washed with 1M aq. NaOH (2 × 750 mL), followed by brine (750 mL), dried over Na₂SO₄, filtered and concentrated under reduced pressure to afford the crude product as an off-white solid. This material was purified by silica gel chromatography eluting with 80-98% EtOAc/Pet. The fractions containing the desired product were collected and concentrated under reduced pressure to afford (S)-N-(7-(2-(1-amino-2-(3,5-difluorophenyl)ethyl)-7-(4-(difluoromethyl)pyrimidin-2-yl)-4-oxoquinazolin-3(4H)-yl)-4-chloro-1-methyl-1H-indazol-3-yl)methanesulfonamide as a yellow solid, 65 g (74%). The product is a mixture of homochiral atropisomers (diastereomers). The above procedure was repeated an addition four times to produce in total 310 g of (S)-N-(7-(2-(1-amino-2-(3,5-difluorophenyl)ethyl)-7-(4-(difluoromethyl)pyrimidin-2-yl)-4-oxoquinazolin-3(4H)-yl)-4-chloro-1-methyl-1H-indazol-3-yl)methanesulfonamide which was dissolved in DCM:MeCN (30:70, 3055 mL) and then subjected to prep-SFC using the following method: Column = (R,R) Welk-01, 30 x 250 mm, 5µ; Eluent = CO₂:methanol (1:1); Flow-rate = 90.0 g/min.; Back-pressure = 120.0 bar; Detection = 254 nm (UV); Stack time = 16.0 min.; Load per injection = 800 mg. The separation produced two peaks. The major peak (second to elute) was collected and concentrated under reduced pressure to afford (S)-N-((6P)-7-(2-(1-amino-2-(3,5-difluorophenyl)ethyl)-7-(4-(difluoromethyl)pyrimidin-2-yl)-4-oxoquinazolin-3(4H)-yl)-4-chloro-1-methyl-1H-indazol-3-yl)methanesulfonamide as a yellow solid, 170 g (51%). The product is a single stereoisomer. ¹H NMR (400 MHz, CDCl₃) *δ* = 9.16-9.07 (m, 1H), 9.01-8.95 (m, 1H), 8.71-8.62 (m, 1H), 8.46-8.37 (m, 1H), 7.68-7.61 (m, 1H), 7.11 (d, *J=* 7.8 Hz, 1H), 6.86-6.55 (m, 2H), 6.54-6.45 (m, 3H), 3.79-3.74 (m, 3H), 3.71-3.63 (m, 1H), 3.44-3.33 (m, 4H), 2.94-2.83 (m, 1H). LCMS Purity = 94%.

### Step 3: Preparation of N-((S)-1-((3P)-3-(4-chloro-1-methyl-3-(methylsulfonamido)-1H-indazol-7-yl)-7-(4-(difluoromethyl)pyrimidin-2-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

To a solution of (S)-N-((6P)-7-(2-(1-amino-2-(3,5-difluorophenyl)ethyl)-7-(4-(difluoromethyl)pyrimidin-2-yl)-4-oxoquinazolin-3(4H)-yl)-4-chloro-1-methyl-1H-indazol-3-yl)methanesulfonamide (50 g, 61.9 mmol), 2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetic acid (16.34 g, 61.9 mmol) and 1-hydroxybenzotriazole hydrate ("HOBt hydrate", 3.79 g, 24.74 mmol) in DMF (500 mL) at 27 °C was added N-methylmorpholine (13.60 mL, 124 mmol) and N-(3-dimethylaminopropyl)-N'-ethylcarbodiimide hydrochloride (21.34 g, 111 mmol). The reaction mixture was stirred at 27 °C for 16 h. The progress of the reaction was monitored by TLC (SiO₂, 50% EtOAc/Pet., Rf = 0.5, UV-active). The reaction mixture was diluted with ice cold water (7 L) and then stirred for 30 min. The precipitated solid was collected via filtration and was then dried under vacuum to afford the crude compound as an off-white solid, 75 g. LCMS purity = 60%. The above procedure was repeated an additional three times to produce in total 185 g of crude N-((S)-1-((3P)-3-(4-chloro-1-methyl-3-(methylsulfonamido)-1H-indazol-7-yl)-7-(4-(difluoromethyl)pyrimidin-2-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide which was blended and then purified silica gel chromatography eluting with 30-40% EtOAc/Pet. The fractions containing the desired product were collected and concentrated under reduced pressure to afford the desired product as an off-white solid (100 g, LCMS Purity: 97%). This material was suspended in isopropanol (1000 mL, 10V), heated at 70 °C for 30 min, then allowed to slowly cool to 27 °C over 16 h to produce the crystalline product. The obtained solid was collected via filtration and then dried under vacuum to afford N-((S)-1-((3P)-3-(4-chloro-1-methyl-3-(methylsulfonamido)-1H-indazol-7-yl)-7-(4-(difluoromethyl)pyrimidin-2-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide as an off-white solid, 80g (79%). 1H NMR (acetone-d6) δ: 9.27 (d, J=5.1 Hz, 1H), 8.89 (d, J=1.8 Hz, 1H), 8.69 (dd, J=8.3, 1.8 Hz, 1H), 8.57 (br s, 1H), 8.39 (d, J=8.3 Hz, 1H), 8.12 (d, J=8.9 Hz, 1H), 7.84 (d, J=5.1 Hz, 1H), 7.51 (d, J=7.7 Hz, 1H), 7.37 (d, J=8.0 Hz, 1H), 7.03 (t, J=54.4 Hz, 1H), 6.86 (tt, J=9.2, 2.4 Hz, 1H), 6.70-6.76 (m, 2H), 6.78 (t, J=54.7 Hz, 1H), 4.93 (td, J=9.0, 4.6 Hz, 1H), 4.65-4.76 (m, 2H), 3.69 (s, 3H), 3.56 (dd, J=14.2, 4.6 Hz, 1H), 3.27 (s, 3H), 3.15 (dd, J=14.3, 9.2 Hz, 1H), 2.42-2.53 (m, 2H), 1.37-1.44 (m, 1H), 0.95-1.00 (m, 1H). LCMS Method: Column = Acquity BEH C18, 2.1 x 50 mm, 1.7 µm particles; Solvent A = 0.1% Formic acid in Water; Solvent B = 0.1% Formic Acid in Acetonitrile; Flow Rate = 0.6 mL/min.; Gradient {time-point (min.) / %B at time-point (%)} = 0/3, 0.4/3, 3.2/98, 3.8/98, 4.2/3, 4.5/3; Column Temperature = 35 °C. LCMS Result: retention time = 2.73 mins.; observed ion = 933.09 (M+H); purity = 99%.

### Naming of Example 2:

The compound of Example 2 as prepared above is a homochiral material that contains axial chirality. Axial chirality can be described using P/M nomenclature as detailed in the IUPAC Gold Book (doi:10.1351/goldbook.A00547). However, at this time only a limited number of software tools are available which are capable of generating chemical names containing P/M nomenclature, and even fewer options are available to convert chemical names using this nomenclature to structural representations of the molecules. Therefore, for clarity and convenience several names for Example 2 are provided below:
The name of Example 2 as generated by ChemDraw Professional 16 (absent P/M nomenclature) is:
   N-((S)-1-(3-(4-chloro-1-methyl-3-(methylsulfonamido)-1H-indazol-7-yl)-7-(4-(difluoromethyl)pyrimidin-2-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide
The chemical name of Example 2 as generated by JChem for Excel (including P/M nomenclature) is:
   N-[(1S)-1-[(3P)-3-(4-chloro-3-methanesulfonamido-1-methyl-1H-indazol-7-yl)-7-[4-(difluoromethyl)pyrimidin-2-yl]-4-oxo-3,4-dihydroquinazolin-2-yl]-2-(3,5-difluorophenyl)ethyl]-2-[(2S,4R)-9-(difluoromethyl)-5,5-difluoro-7,8-diazatricyclo[4.3.0.0²,⁴]nona-1(6),8-dien-7-yl]acetamide
The chemical name of Example 2 generated by ChemDraw Professional 16 with manually added P/M nomenclature is:
   N-((S)-1-((3P)-3-(4-chloro-1-methyl-3-(methylsulfonamido)-1H-indazol-7-yl)-7-(4-(difluoromethyl)pyrimidin-2-yl)-4-oxo-3,4-dihydroquinazolin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

### Preparation of 3,3-difluorobutan-1-ol

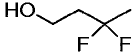

### Synthesis Scheme:

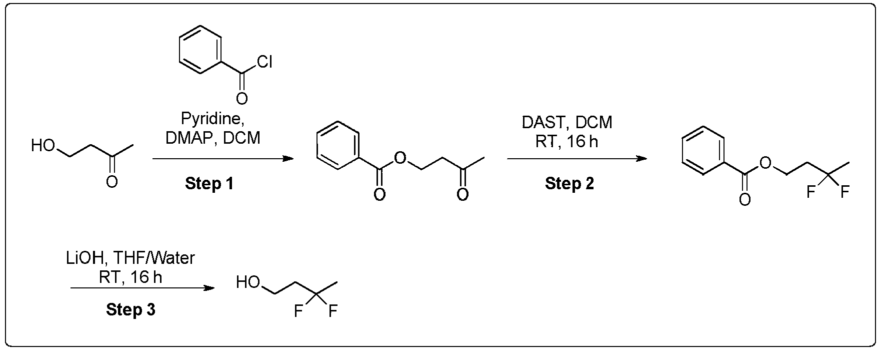

### Step 1: Preparation of 3-oxobutyl benzoate

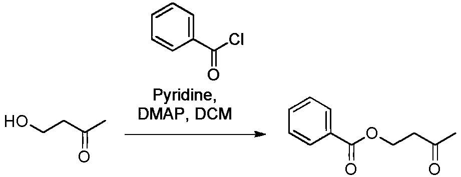

To a stirred solution of benzoyl chloride (0.396 L, 3405 mmol) in DCM (1 L) at -70 °C under nitrogen atmosphere was added pyridine (470 mL) drop-wise over a period of 1 h. After stirring for 30 min at same temperature, 4-hydroxybutan-2-one (250.0 g, 2837 mmol) in DCM (500 mL) was added dropwise over a period of 1 h. The reaction mixture was allowed to warm to 26 °C and then was stirred for 16 h. The progress of the reaction was monitored by TLC (SiO₂, 30% EtOAc/Pet. Rf = 0.4). On completion, the reaction mixture was washed with water (2 x 1000 mL), 1N HCl (2 x 500 mL) and then saturated NaHCO₃ solution (2 x 500 mL). The organic layer was dried over Na₂SO₄, filtered and concentrated under reduced pressure to afford 3-oxobutyl benzoate as a pale-yellow liquid (400 g, yield = 69%). ¹H NMR (400 MHz, CHLOROFORM-d) δ = 8.05 - 7.94 (m, 2H), 7.60 - 7.51 (m, 1H), 7.47 - 7.36 (m, 2H), 4.65 - 4.54 (t, 2H), 2.97 - 2.84 (t, 2H), 2.28 - 2.13 (s, 3H). HPLC purity = 94.1%.

### Step 2: Preparation of 3,3-difluorobutyl benzoate

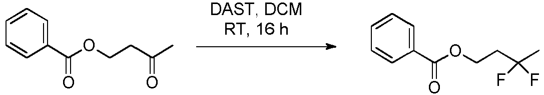

To the stirred solution of 3-oxobutyl benzoate (90 g, 427 mmol) in dichloromethane (700 mL) at 0 °C under nitrogen atmosphere was added DAST (677 mL, 5125 mmol) dropwise over a period of 1 h. The reaction mixture was warmed to 26 °C and stirred for 16 hr. The progress of reaction was monitored by TLC (SiO₂, 20% EtOAc/Pet. Rf = 0.6). On completion, the reaction mixture was diluted with DCM (500 mL) and slowly poured into cold aq. saturated NaHCO₃ (1 L) solution. The organic layer was separated and washed with brine solution (400 mL), dried over anhydrous Na₂SO₄, filtered and concentrated under reduced pressure to afford the crude compound as a yellow liquid (95 g). This material was purified by column chromatography using silica gel (100-200 mesh), eluted with 0-5% EtOAc in pet. The fractions containing product were collected and concentrated under reduced pressure to afford 3,3-difluorobutyl benzoate as a brown liquid (60 g, yield = 59%,). ¹H NMR (400 MHz, CDCl₃) δ = 8.06 - 8.01 (m, 2H), 7.60 - 7.54 (m, 1H), 7.48 - 7.40 (m, 2H), 4.54 - 4.48 (t, 2H), 2.43 - 2.29 (m, 2H), 1.77 - 1.64 (m, 3H). LCMS purity = 89.74%; m/z = 215.33.

### Step 3: Preparation of 3,3-difluorobutan-1-ol

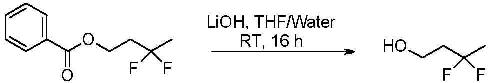

To a stirred solution of 3,3-difluorobutyl benzoate (100 g, 467 mmol) in THF (800 mL) at 0 °C under nitrogen atmosphere was added a solution of lithium hydroxide monohydrate (137 g, 3268 mmol) in water (800 mL). The reaction mixture was allowed to warm to 26 °C and then was stirred for 16 hr. The progress of the reaction was monitored by TLC (SiO₂, 20% EtOAc/Pet. Rf = 0.6, KMnO₄ active). On completion, the reaction mixture was diluted with diethyl ether (400 mL). The organic layer was separated and the aqueous layer was again extracted with diethyl ether (300 mL). The combined organics were washed with brine (200 mL), dried over Na₂SO₄, filtered and concentrated under reduced pressure (volatile product, bath temperature = 25°C) to afford the crude compound as a black liquid. This material was diluted with diethyl ether (100 mL) and treated with charcoal. The mixture was filtered through a pad of Celite. The Celite pad was extracted with diethyl ether (200 mL). The combined filtrates were concentrated under reduced pressure (volatile product, bath temperature = 25°C) to afford 3,3-difluorobutan-1-ol as a pale-yellow liquid (40 g, yield =71%). ¹H-NMR (400 MHz, CDCl₃) δ = 3.87 (t, *J=* 6.1 Hz, 2H), 2.22 - 2.07 (m, 2H), 1.73 - 1.57 (m, 3H). GCMS Purity = 91.3%; m/z = 110.0.

### Preparation of 2-amino-6-(benzyloxy) nicotinic acid

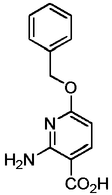

### Synthesis Scheme:

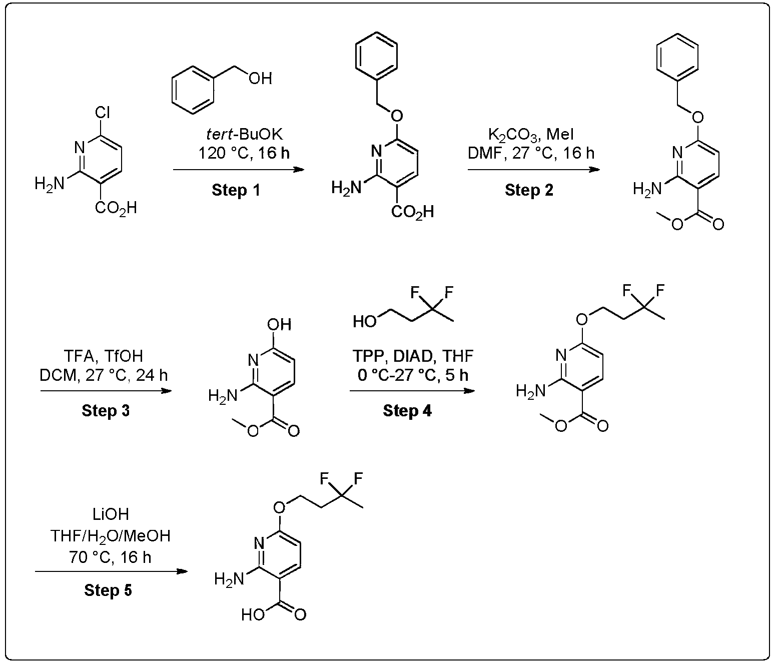

### Step 1: Preparation of 2-amino-6-(benzyloxy) nicotinic acid

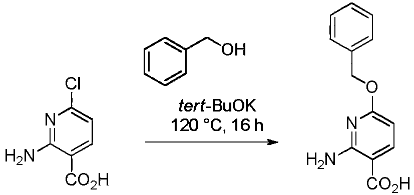

To a stirred solution of 2-amino-6-chloronicotinic acid (200 g, 1159 mmol) in benzyl alcohol (1400 mL, 13464 mmol) at 26 °C under N₂ atmosphere was added potassium tertbutoxide (390 g, 3477 mmol). The reaction mixture was heated to 120 °C and stirred for 16 hr at that temperature. The progress of reaction was monitored by TLC (SiO₂, 10% MeOH in DCM, Rf = 0.5). On completion, the reaction mixture was diluted with water (3 L) and extracted with diethyl ether (2 x 1000 mL). The organic layer was separated and the aqueous layer was acidified to pH 4 using aq. citric acid solution (0.5 M). The precipitated solid was collected by filtration and then dried under reduced pressure to afford 2-amino-6-(benzyloxy) nicotinic acid as an off-white solid (220 g, yield = 72%). ¹H NMR (400 MHz, DMSO-d₆) δ = 12.56 - 12.32 (m, 1H), 7.97 - 7.91 (m, 1H), 7.52 - 7.41 (m, 2H), 7.38 - 7.11 (m, 5H), 6.03 (d, *J=* 8.5 Hz, 1H), 5.39 - 5.31 (m, 2H). LCMS Purity = 93%; m/z = 245.29 (M+H).

### Step 2: Preparation of methyl 2-amino-6-(benzyloxy)nicotinate

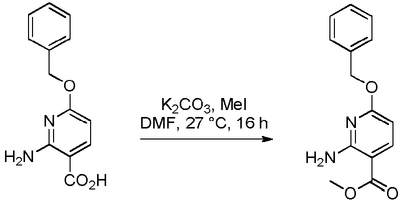

To a stirred solution of 2-amino-6-(benzyloxy)nicotinic acid (220 g, 901 mmol) in DMF (2.5 L) at 26 °C under N₂ atmosphere were slowly added potassium carbonate (373 g, 2702 mmol) and iodomethane (0.282 L, 4504 mmol). The reaction mixture was stirred at 27 °C for 16 hr. The progress of reaction was monitored by TLC (SiO₂, 40% EtOAc/Pet., Rf = 0.6). On completion, the reaction mixture was diluted with water (5 L). The precipitated solid was isolated by filtration and then dried under vacuum to afford methyl 2-amino-6-(benzyloxy)nicotinate as an off-white solid (220 g, yield= 92 %). ¹H NMR (400 MHz, CDCl₃) *δ* = 8.00 (d, *J=* 8.4 Hz, 1H), 7.42-7.40 (m, 2H), 7.39-7.35 (m, 2H), 7.34-7.31 (m, 1H), 6.01 (d, *J=* 8.4 Hz, 1H), 5.33 (s, 2H), 3.84 (s, 3H). LCMS Purity = 97%, m/z = 259.30 (M+H).

### Step 3: Preparation of methyl 2-amino-6-hydroxynicotinate

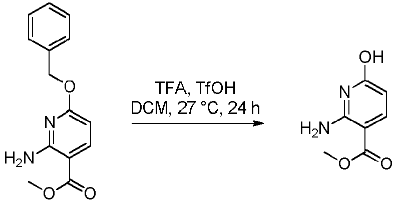

To a stirred solution of methyl 2-amino-6-(benzyloxy)nicotinate (50 g, 190 mmol) in DCM (500 mL) at 26 °C under N₂ atmosphere were slowly added TFA (800 mL) and triflic acid (25 mL, 282 mmol). The reaction mixture was stirred at 26 °C for 16 hr. The progress of reaction was monitored by TLC (SiO₂, EtOAc, Rf = 0.2). On completion, the volatiles were removed under vacuum to afford the crude product. This material was triturated with diethyl ether (3 x 1000 mL) and the precipitated solid was then isolated by filtration. To the solid was added water (2 L) and the mixture was then 5 h. The solid was collected by filtration and was washed with water. The solid was dried under vacuum to afford methyl 2-amino-6-hydroxynicotinate as an off-white solid (25 g, yield = 78%). 1H NMR (300 MHz, DMSO-d₆) *δ* = 10.92-10.76 (m, 1H), 7.65 (d, *J* = 9.5 Hz, 1H), 7.43-6.87 (m, 2H), 5.51 (d, *J* = 9.5 Hz, 1H), 3.69 (s, 3H). LCMS Purity = 99.32%; m/z = 169.32 (M+H). The absence of TFA and triflic acid in the product was confirmed by ¹⁹F-NMR. The product was used directly in the next step without additional purification.

### Step 4: Preparation of methyl 2-amino-6-(3,3-difluorobutoxy)nicotinate

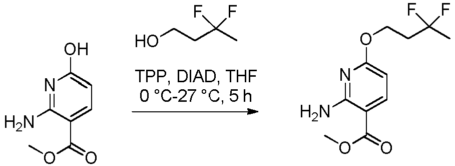

To a stirred solution of methyl 2-amino-6-hydroxynicotinate (25 g, 147 mmol) in THF (375 mL) at 0 °C under N₂ atmosphere were added triphenylphosphine (77 g, 294 mmol) and then drop-wise DIAD (57.2 mL, 294 mmol). The reaction mixture was stirred for 15 min at 0 °C, then to the mixture was added drop-wise at 0 °C a solution of 3,3-difluorobutan-1-ol (25.3 g, 221 mmol) in THF (125 mL). The reaction mixture was allowed to 27 °C and then was stirred for 5 h. The progress of reaction was monitored by TLC (SiO₂, EtOAc, Rf = 0.5). On completion, the reaction mixture was concentrated under reduced pressure to afford the crude product. This material was stirred in MTBE:pet. (1:1, 1 L). The mixture was filtered and the filter pad was extracted with MTBE:pet. (1:1, 4 x 200 mL). The combined filtrate was concentrated under reduced pressure to afford a pale-yellow gummy solid. This material was purified by column chromatography using silica gel (100-200 mesh), eluted with 10-20% EtOAc in pet. The fractions containing product were collected and concentrated under reduced pressure to afford methyl 2-amino-6-(3,3-difluorobutoxy)nicotinate as a pale-yellow liquid (20 g, yield = 48%. ¹H NMR (400 MHz, CDCl₃) δ = 8.05 - 7.95 (m, 1H), 6.02 (d, *J=* 8.8 Hz, 1H), 4.45 (t, *J* = 6.8 Hz, 2H), 3.80 (s, 3H), 2.40 - 2.22 (m, 2H), 1.68 (t, *J* = 18.6 Hz, 3H). LCMS purity = 91.1%, m/z = 261.25 (M+H).

### Step 5: Preparation of 2-amino-6-(3,3-difluorobutoxy)nicotinic acid

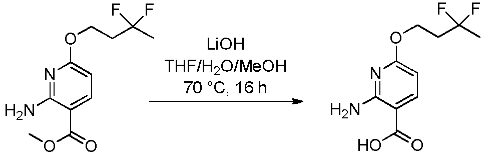

To a stirred solution of methyl 2-amino-6-(3,3-difluorobutoxy)nicotinate (5.7g, 20.81 mmol) in THF (120 mL) and Methanol (30 mL) at 26 °C was added a solution of LiOH (2.491 g, 104 mmol) in water (30 mL). The reaction mixture was heated to 70 °C and stirred at that temperature for 16 h. The progress of reaction was monitored by TLC (SiO₂, 50% EtOAc/Pet Rf = 0.2). On completion, the reaction mixture was concentrated under reduced pressure. The resulting residue was dissolved in water (60 mL) and acidified to pH 4 using 1N HCl. The mixture was extracted with ethyl acetate (3 x 100 mL). The combined organics were washed with brine (100 mL), dried over anhydrous Na₂SO₄, and concentrated under reduced pressure to afford 2-amino-6-(3,3-difluorobutoxy)nicotinic acid as a brown solid (4.6 g, yield = 87%). ¹H NMR (400 MHz, CDCl₃) δ = 11.66 - 10.84 (m, 1H), 8.12 - 7.97 (m, 1H), 6.07 (d, *J=* 8.3 Hz, 1H), 4.52 - 4.36 (m, 2H), 2.41 - 2.28 (m, 2H), 1.68 (t, *J* = 18.6 Hz, 3H). LCMS Purity = 97.68%, m/z = 247.24 (M+H).

### Preparation of Example 3: N-((S)-1-((3P)-3-(4-chloro-1-methyl-3-(methylsulfonamido)-1H-indazol-7-yl)-7-(3,3-difluorobutoxy)-4-oxo-3,4-dihydropyrido[2,3-d]pyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

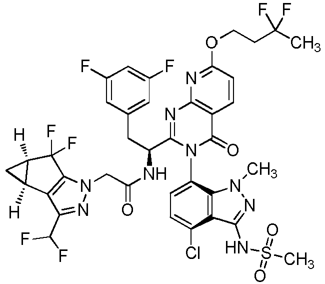

### Synthesis Scheme:

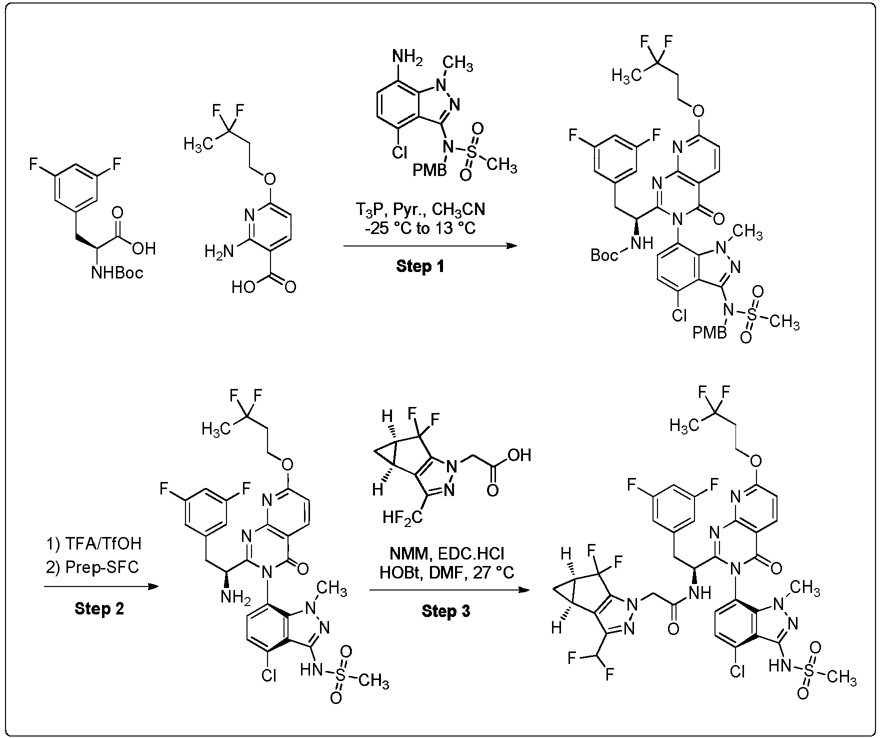

### Step 1: Preparation of tert-Butyl (S)-(1-(3-(4-chloro-3-(N-(4-methoxybenzyl)methylsulfonamido)-1-methyl-1H-indazol-7-yl)-7-(3,3-difluorobutoxy)-4-oxo-3,4-dihydropyrido[2,3-d]pyrimidin-2-yl)-2-(3,5-difluorophenyl)ethyl)carbamate

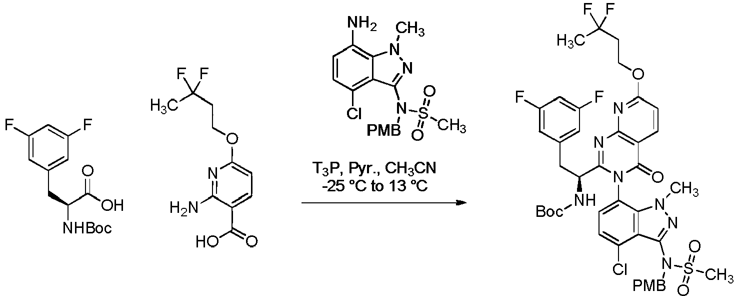

To a stirred solution of (S)-2-((tert-butoxycarbonyl)amino)-3-(3,5-difluorophenyl)propanoic acid (50 g, 166 mmol) and 2-amino-6-(3,3-difluorobutoxy)nicotinic acid (41.3 g, 166 mmol) in acetonitrile (1000 mL) under nitrogen atmosphere at -25 °C was added pyridine (47.0 mL, 581 mmol). To the resulting mixture was added 2,4,6-tripropyl-1,3,5,2,4,6-trioxatriphosphinane 2,4,6-trioxide ("T3P", 50% wt in EtOAc, 494 mL, 830 mmol) drop-wise over 15 min. The solution was allowed to warm to 13 °C and was then stirred for 5 hrs. To the solution at 13 °C was added N-(7-amino-4-chloro-1-methyl-1H-indazol-3-yl)-N-(4-methoxybenzyl)methanesulfonamide (62.3 g, 158 mmol). The reaction mass was then allowed to slowly warm to 27 °C and then was stirred at that temperature for 48 hrs. The progress of the reaction was monitored by TLC (SiO₂, 50% EtOAc/Pet., Rf = 0.4). On completion, the reaction mixture was concentrated under reduced pressure and the residue was added dropwise into saturated aqueous NaHCO₃ solution (1000 mL) at 0 °C. A white precipitate was formed which was collected by vacuum filtration. The isolated solids were washed with water (2 L). Vacuum filtration was maintained until most residual water had been removed from the solids. The solids were then dissolved in DCM (2 L). The solution was dried over Na₂SO₄, filtered and then concentrated under reduced pressure to afford the crude product. This material was purified by silica gel chromatography eluting with 50-65% EtOAc in Pet. The fractions containing the desired product were pooled and concentrated under reduced pressure to afford tert-butyl (S)-(1-(3-(4-chloro-3-(N-(4-methoxybenzyl)methylsulfonamido)-1-methyl-1H-indazol-7-yl)-7-(3,3-difluorobutoxy)-4-oxo-3,4-dihydropyrido[2,3-d]pyrimidin-2-yl)-2-(3,5-difluorophenyl)ethyl)carbamate as a yellow foamy solid (50 g, Yield = 31%). The above procedure was repeated seven more times on the same scale to produce in total 592 g of the product. The combined product (592 g) was dissolved in MeOH (1 L). The solution was diluted with n-hexane (6 L). An off-white solid precipitated and then suspension was stirred for 20 min. The solid was collected by vacuum filtration while the filtrate was reserved. The solid was dried under vacuum to afford tert-butyl (S)-(1-(3-(4-chloro-3-(N-(4-methoxybenzyl)methylsulfonamido)-1-methyl-1H-indazol-7-yl)-7-(3,3-difluorobutoxy)-4-oxo-3,4-dihydropyrido[2,3-d]pyrimidin-2-yl)-2-(3,5-difluorophenyl)ethyl)carbamate as an off-white solid (300 g, Yield = 48%) as an off-white solid. The product is a mixture of homochiral atropisomers (diastereomers). LCMS analysis method: Column = X Bridge BEH C18 (50mm x 4.6mm, 2.5 µm particles); Mobile Phase A = 5mM Ammonium Bicarbonate; Mobile Phase B = acetonitrile; Gradient profile (time (min) / %B) = 0/5, 0.5/5, 1.5/15, 7/98, 9/98, 9.5/5, 10/5; Column Temp = 35°C; Flow rate = 1.3 mL/min. LCMS result: retention time = 6.20 mins.; observed ion = 888.09 (M+H); LCMS Purity = 95%. Note: The reserved filtrate was concentrated and dried under vacuum to afford the product (120 g, a pale yellow solid) which was also used in downstream chemistry separately from the product described above.

### Step 2: Preparation of (S)-N-((6P)-7-(2-(1-amino-2-(3,5-difluorophenyl)ethyl)-7-(3,3-difluorobutoxy)-4-oxopyrido[2,3-d]pyrimidin-3(4H)-yl)-4-chloro-1-methyl-1H-indazol-3-yl)methanesulfonamide

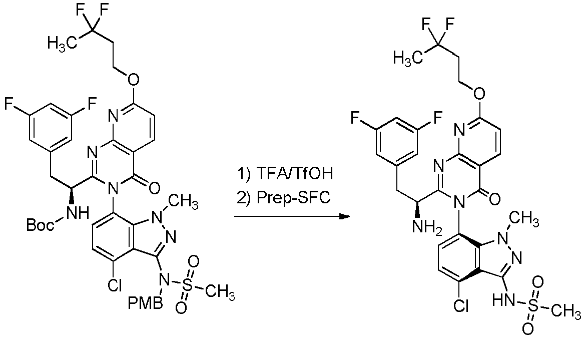

To a stirred solution of tert-butyl (S)-(1-(3-(4-chloro-3-(N-(4-methoxybenzyl)methylsulfonamido)-1-methyl-1H-indazol-7-yl)-7-(3,3-difluorobutoxy)-4-oxo-3,4-dihydropyrido[2,3-d]pyrimidin-2-yl)-2-(3,5-difluorophenyl)ethyl)carbamate (95% purity, 300 g, 321 mmol) in DCM (3000 mL) were added at 0 °C trifluoroacetic acid (TFA) (900 mL) followed by triflic acid (158 mL, 1782 mmol). The solution was allowed to warm to 27 °C and was then stirred for 2 hrs under nitrogen atmosphere. The progress of the reaction was monitored by TLC (SiO₂, 80% EtOAc/Pet. Rf = 0.3). On completion, the volatiles were removed under a gentle stream of nitrogen gas. The residue was added into saturated NaHCO₃ solution (1000 mL) at 0 °C. The was adjusted to solution pH ~8 by addition of solid NaHCO₃. The mixture was extracted with EtOAc (5 × 1000 mL). The combined organic layers were dried over Na₂SO₄, filtered, and then concentrated under reduced pressure to afford the crude product. This material was purified by silica gel chromatography eluting with 5-10% MeOH in DCM. The fractions containing the desired product were pooled and concentrated under reduced pressure to afford (S)-N-(7-(2-(1-amino-2-(3,5-difluorophenyl)ethyl)-7-(3,3-difluorobutoxy)-4-oxopyrido[2,3-d]pyrimidin-3(4H)-yl)-4-chloro-1-methyl-1H-indazol-3-yl)methanesulfonamide (211 g, brown foamy solid) as a mixture of homochiral atropisomers (diastereomers, Major: 79% and Minor: 10% by LCMS). The material was dissolved in methanol:acetonitrile (80:20, 1800 mL) and was then purified by prep-SFC using the following method: Column = (R,R) WHELK-01 (30 x 250 mm, 5 µm particles); eluent = CO₂:MeOH (60:40); Flow-rate = 90 g/min; Back-pressure = 100 bar; Detection = 214 nm (UV); Stack time = 15.5 min; Load per injection = 1.125 gram. The pure major peak was collected and concentrated under reduced pressure to afford (S)-N-((6P)-7-(2-(1-amino-2-(3,5-difluorophenyl)ethyl)-7-(3,3-difluorobutoxy)-4-oxopyrido[2,3-d]pyrimidin-3(4H)-yl)-4-chloro-1-methyl-1H-indazol-3-yl)methanesulfonamide (151 g, Yield = 69%) as a brown solid. The product is a single stereoisomer. 1H-NMR (400 MHz, DMSO-d₆) *δ*: 8.41 (d, *J* = 8.8 Hz, 1H), 7.39 (dd, *J=* 22.4, 7.9 Hz, 2H), 7.05 (d, *J=* 8.3 Hz, 1H), 7.03-6.98 (m, 1H), 6.72 (d, *J=* 8.8 Hz, 2H), 4.66-4.63 (m, 2H), 3.67 (s, 3H), 3.54-3.50 (m, 1H), 3.28-3.23 (m, 1H), 3.21 (s, 3H), 2.88-2.82 (m, 1H), 2.56-2.52 (m, 1H), 2.47-2.44 (m, 1H), 1.73 (t, *J=* 19.0 Hz, 3H); LCMS method: Column = Acquity BEH C18 (50mm x 2.1mm, 1.7 µm particles); Mobile Phase A = 0.1% Formic Acid in water; Mobile Phase B = 0.1% Formic Acid in MeCN; Gradient profile (time (min) / %B): 0/3, 0.4/3, 3.2/98, 3.8/98, 4.2/3, 4.5/3; Column Temp = 35°C; Flow rate: 0.6 mL/min. LCMS result: retention time = 1.93 mins.; observed ion = 668.05 (M+H); HPLC Purity = 98%; Chiral HPLC Purity = 96.9%.

### Step 3: Preparation of N-((S)-1-((3P)-3-(4-chloro-1-methyl-3-(methylsulfonamido)-1H-indazol-7-yl)-7-(3,3-difluorobutoxy)-4-oxo-3,4-dihydropyrido[2,3-d]pyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

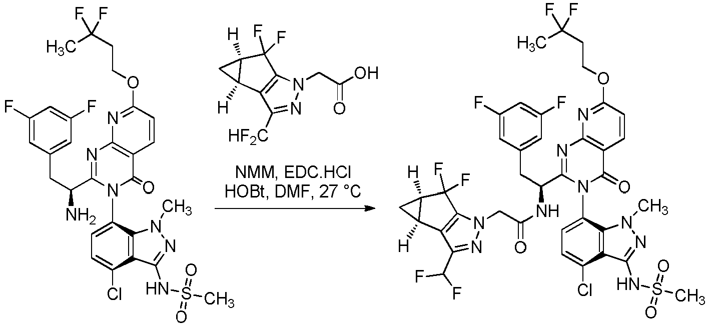

To a stirred solution of (S)-N-((6P)-7-(2-(1-amino-2-(3,5-difluorophenyl)ethyl)-7-(3,3-difluorobutoxy)-4-oxopyrido[2,3-d]pyrimidin-3(4H)-yl)-4-chloro-1-methyl-1H-indazol-3-yl)methanesulfonamide (50 g, 74.1 mmol) in DMF (500 mL) at 27 °C was added 2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetic acid (21.75 g, 82 mmol) followed by N-(3-dimethylaminopropyl)-N'-ethylcarbodiimide hydrochloride ("EDC-HCl", 18.47 g, 96 mmol), 1-hydroxybenzotriazole hydrate ("HOBt hydrate", 13.62 g, 89 mmol), and N-methylmorpholine (65.2 mL, 593 mmol). The reaction mass was stirred for 16 hr at 27 °C. The progress of the reaction was monitored by TLC (SiO₂, 50% EtOAc/Pet., Rf = 0.5). On completion, the reaction mass was diluted with ice water (1 L) and the resulting precipitate was collected via filtration and then dried under vacuum to afford the crude product (77 g) as an off-white solid. This crude product was blended with two additional batches of crude product generated by repeating the procedure on the same scale. Together, the 227 g of crude product was purified by silica gel chromatography eluting with 40-50% EtOAc in Pet. The fractions containing the desired product were pooled and concentrated under reduced pressure to afford the purified product (180 g). This purified product was blended with an additional batch of product (25 g) prepared similarly. A portion of the purified product (150 g) was further purified batch-wise (30 x 5 g) by reverse phase chromatography using the following method: Column = RediSep 275 g, HP C18 (CV 243mL, 150 mL/min); Mobile Phase A = Water:MeCN:TFA (950:50:1); Mobile Phase B = Water:MeCN:TFA (50:950:1); Gradient profile (Time (min) /% B) = 3/10, 6/20, 9/30, 12/40, 15/50, 18/60, 42/70 (compounds start to elute), 52/80, 57/100; flow rate = 80 mL/min.; Column temp = 26 °C; Loading = 5 g each time. The fractions containing the pure product were pooled and concentrated under reduced pressure to remove the acetonitrile component. The aqueous solution was made basic by the addition of saturated NaHCO₃, then was extracted with EtOAc (3 × 500 mL). The combined organics were dried over anhydrous Na₂SO₄ and then filtered. The filtrate was concentrated under reduced pressure to obtain the desired product (102 g) as an off-white solid. This material was dissolved in EtOAc (200 mL) and the solution was then diluted with n-hexane (1 L). The resulting precipitate was stirred for 2 h at 27 °C and then was collected via filtration. The solid was dried under vacuum. Trace solvent residues were removed by grinding the compound using a mortar and pestle and then maintaining the fine solids in a 50 °C oven for approximately 2 h; this process of grinding and heating was repeated (~4-5 times) until all trace solvents were removed (analysed by NMR) to afford N-((S)-1-((3P)-3-(4-chloro-1-methyl-3-(methylsulfonamido)-1H-indazol-7-yl)-7-(3,3-difluorobutoxy)-4-oxo-3,4-dihydropyrido[2,3-d]pyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide as an off-white solid (88.7 g, Yield = 87%). 1H-NMR (DMSO-d₆) δ: 9.86 (s, 1H), 9.45 (d, *J* = 8.3 Hz, 1H), 8.44 (d, *J=* 8.7 Hz, 1H), 7.77 (d, *J* = 8.3 Hz, 1H), 7.48 (d, *J* = 7.4 Hz, 1H), 7.09 (d, *J=* 8.7 Hz, 2H), 7.07-6.77 (m, 1H), 6.65 (d, *J* = 6.2 Hz, 2H), 4.70 (d, *J=* 16.7 Hz, 1H), 4.65 (t, *J=* 6.3 Hz, 2H), 4.55 (d, *J* = 16.7 Hz, 1H), 4.51-4.45 (m, 1H), 3.50 (s, 3H), 3.42-3.37 (m, 1H), 3.18 (s, 3H), 3.06-3.00 (m, 1H), 2.56-2.52 (m, 2H), 2.47-2.42 (m, 2H), 1.73 (t, *J=* 19.2 Hz, 3H), 1.38-1.32 (m, 1H), 0.85-0.81 (m, 1H); LCMS method: Column = Acquity BEH C18 (50mm x 2.1mm, 1.7 µm particles), Mobile Phase A = 0.1% Formic Acid in water; Mobile Phase B = 0.1% Formic Acid in MeCN; Gradient profile (Time (min) / %B) = 0/3, 0.4/3, 7.5/98, 9.5/98, 9.6/3, 10/3; Column temp = 35 °C; Flow rate = 0.6 mL/min. LCMS result: retention time = 5.05 mins.; observed ion = 913.97 (M+H); HPLC Purity = 99.5%; Chiral HPLC Purity = 99.5%.

### Naming of Example 3:

The compound of Example 3 as prepared above is a homochiral material that contains axial chirality. Axial chirality can be described using P/M nomenclature as detailed in the IUPAC Gold Book (doi:10.1351/goldbook.A00547). However, at this time only a limited number of software tools are available which are capable of generating chemical names containing P/M nomenclature, and even fewer options are available to convert chemical names using this nomenclature to structural representations of the molecules. Therefore, for clarity and convenience several names for Example 3 are provided below:
The name of Example 3 as generated by ChemDraw Professional 16 (absent P/M nomenclature) is:
   N-((S)-1-(3-(4-chloro-1-methyl-3-(methylsulfonamido)-1H-indazol-7-yl)-7-(3,3-difluorobutoxy)-4-oxo-3,4-dihydropyrido[2,3-d]pyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide
The chemical name of Example 3 as generated by JChem for Excel (including P/M nomenclature) is:
   N-[(1S)-1-[(3P)-3-(4-chloro-3-methanesulfonamido-1-methyl-1H-indazol-7-yl)-7-(3,3-difluorobutoxy)-4-oxo-3H,4H-pyrido[2,3-d]pyrimidin-2-yl]-2-(3,5-difluorophenyl)ethyl]-2-[(2S,4R)-9-(difluoromethyl)-5,5-difluoro-7,8-diazatricyclo[4.3.0.0²,⁴]nona-1(6),8-dien-7-yl]acetamide
The chemical name of Example 3 generated by ChemDraw Professional 16 with manually added P/M nomenclature is:
   N-((S)-1-((3P)-3-(4-chloro-1-methyl-3-(methylsulfonamido)-1H-indazol-7-yl)-7-(3,3-difluorobutoxy)-4-oxo-3,4-dihydropyrido[2,3-d]pyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

### Preparation of N-[(6P)-7-{2-[(1S)-1-amino-2-(3,5-difluorophenyl)ethyl]-7-hydroxy-4-oxo-3H,4H-pyrido[2,3-d]pyrimidin-3-yl}-4-chloro-1-methyl-1H-indazol-3-yl]-N-[(4-methoxyphenyl)methyl]methanesulfonamide

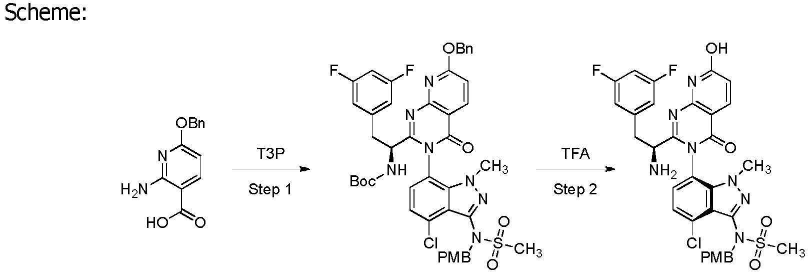

### Step 1:

To a suspension of (S)-2-((tert-butoxycarbonyl)amino)-3-(3,5-difluorophenyl)propanoic acid (5.49 g, 18.23 mmol) and 2-amino-6-(benzyloxy)nicotinic acid (4.45 g, 18.23 mmol) in acetonitrile (92 mL) (yellow solution) at -25 °C was added pyridine (9.83 mL, 122 mmol) followed by 2,4,6-tripropyl-1,3,5,2,4,6-trioxatriphosphinane 2,4,6-trioxide ("T3P", 45.2 ml, 76 mmol). The reaction mixture (became a clear solution after T₃P addition) was stirred at -25 °C to 10 °C over 4.5 h, then N-(7-amino-4-chloro-1-methyl-1H-indazol-3-yl)-N-(4-methoxybenzyl)methanesulfonamide (6 g, 15.19 mmol) was added and the mixture was stirred for 18 h while warming to rt. The reaction mixture was diluted with ethyl acetate, washed with 1N NaOH, then water, then 0.5 M citric acid, then water, then dried over Na₂SO₄ and concentrated in vacuo. The resulting residue was purified on silica (330 g RediSep Gold column) using 0-60 % ethyl acetate in hexanes over 15 CV, then holding at 60% EtOAc for 10 CV. The desired fractions were pooled and concentrated to afford a pale yellow solid (8.1 g, 9.14 mmol, 60.1 % yield), a mixture of tert-butyl N-[(1S)-1-[(3P,3P)-7-(benzyloxy)-3-(4-chloro-3-{N-[(4-methoxyphenyl)methyl]methanesulfonamido}-1-methyl-1H-indazol-7-yl)-4-oxo-3H,4H-pyrido[2,3-d]pyrimidin-2-yl]-2-(3,5-difluorophenyl)ethyl]carbamate (major) and tert-butyl N-[(1S)-1-[(3M,3M)-7-(benzyloxy)-3-(4-chloro-3-{N-[(4-methoxyphenyl)methyl] methanesulfonamido }-1-methyl-1H-indazol-7 -yl)-4-oxo- 3H,4H-pyrido[2,3-d]pyrimidin-2-yl]-2-(3,5-difluorophenyl)ethyl]carbamate (minor). LC/MS: m/z = 886.25 [M+1]⁺.

### Step 2:

TFA (21.1 mL, 274 mmol) was added to a solution of tert-butyl (S)-(1-(7-(benzyloxy)-3-(4-chloro-3-(N-(4-methoxybenzyl)methylsulfonamido)-1-methyl-1H-indazol-7-yl)-4-oxo-3,4-dihydropyrido[2,3-d]pyrimidin-2-yl)-2-(3,5-difluorophenyl)ethyl)carbamate (Product from Step 1, 8.1 g, 9.14 mmol) in dichloromethane (45.7 mL). The mixture was stirred at rt for 2 h. The resultant pale yellow solution was concentrated. The residue was taken up in ethyl acetate, then washed three times with 1 N NaOH, then dried over Na2SO4 and then concentrated in vacuo to afford an oily residue. The residue was purified on silica gel (330 g RediSep Gold column) by a gradient method of Solvent A:Solvent B 65:35→0:100 (2 CV), then 0:100 (9 CV); Solvent A = hexanes; Solvent B = 9:9:2 hexanes:ethyl acetate:MeOH. The first eluting isomer (major) was collected and concentrated in vacuo to afford N-[(6P)-7-{2-[(1S)-1-amino-2-(3,5-difluorophenyl)ethyl]-7-hydroxy-4-oxo-3H,4H-pyrido[2,3-d]pyrimidin-3-yl}-4-chloro-1-methyl-1H-indazol-3-yl]-N-[(4-methoxyphenyl)methyl]methanesulfonamide (4.1 g, 5.89 mmol, 64.5 % yield). ¹H NMR (500 MHz, DMSO-d6) δ 7.86 - 7.98 (m, 1 H) 7.15 - 7.37 (m, 4 H) 6.97 - 7.06 (m, 1 H) 6.70 - 6.89 (m, 4 H) 6.40 - 6.48 (m, 1 H) 4.70 - 4.88 (m, 2 H) 3.41 - 3.81 (m, 7 H) 3.20 - 3.28 (m, 1 H) 3.08 - 3.12 (m, 3 H) 2.71 - 2.79 (m, 1 H) 1.69 - 2.00 (m, 2 H). LC/MS: m/z = 696.20 [M+1]⁺.

### Preparation of N-((S)-1-((3P)-3-(4-chloro-3-(N-(4-methoxybenzyl)methylsulfonamido)-1-methyl-1H-indazol-7-yl)-7-hydroxy-4-oxo-3,4-dihydropyrido[2,3-d]pyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

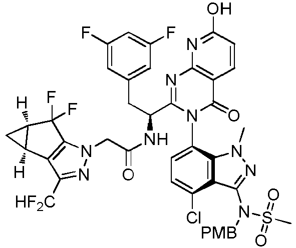

To a stirred solution of N-[(6P)-7-{2-[(1S)-1-amino-2-(3,5-difluorophenyl)ethyl]-7-hydroxy-4-oxo-3H,4H-pyrido[2,3-d]pyrimidin-3-yl}-4-chloro-1-methyl-1H-indazol-3-yl]-N-[(4-methoxyphenyl)methyl]methanesulfonamide (0.926 g, 1.330 mmol) in DMF (13 ml) was added 2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetic acid (0.351 g, 1.330 mmol), 2-(3H-[1,2,3]triazolo[4,5-b]pyridin-3-yl)-1,1,3,3-tetramethylisouronium hexafluorophosphate(V) ("HATU", 0.531 g, 1.397 mmol), and DIPEA (0.581 ml, 3.33 mmol). The reaction mixture was stirred for 2 h after which the reaction mixture was diluted with water and extracted with ethyl acetate. The combined EtOAc extractions were washed with brine, dried over Na₂SO₄, and concentrated in vacuo. The crude product was purified via silica gel flash chromatography using 10-100% ethyl acetate in hexanes to provide N-((S)-1-((3P)-3-(4-chloro-3-(N-(4-methoxybenzyl)methylsulfonamido)-1-methyl-1H-indazol-7-yl)-7-hydroxy-4-oxo-3,4-dihydropyrido[2,3-d]pyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (1.1 g, 88%) as an off-white foamy solid. LC/MS: m/z = 942.25 [M+1]⁺.

### Preparation of Example 4: N-((S)-1-((3P)-3-(4-chloro-1-methyl-3-(methylsulfonamido)-1H-indazol-7-yl)-4-oxo-7-(3,3,3-trifluoropropoxy)-3,4-dihydropyrido[2,3-d]pyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

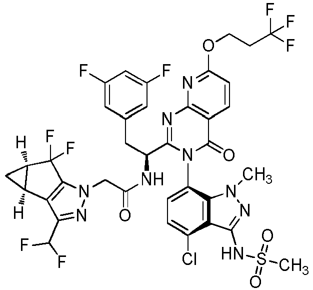

A solution of diisopropyl (E)-diazene-1,2-dicarboxylate ("DIAD", 0.125 ml, 0.637 mmol) in THF (0.2 mL) was added dropwise to a mixture of N-(1-((3P)-3-(4-chloro-3-(N-(4-methoxybenzyl)methylsulfonamido)-1-methyl-1H-indazol-7-yl)-7-hydroxy-4-oxo-3,4-dihydropyrido[2,3-d]pyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (0.2 g, 0.212 mmol)), 3,3,3-trifluoropropan-1-ol (0.073 g, 0.637 mmol) and triphenylphosphine (0.178 g, 0.679 mmol) in Tetrahydrofuran (2.1 mL) at rt. The reaction mixture was stirred for 18 h at rt and then was concentrated *in vacuo.* The residue was purified on silica gel (24 g RediSep Gold column) using a gradient of 0-60 % ethyl acetate in hexanes over 15 CV, and then holding at 60 % ethyl acetate in hexanes for 5 CV. Fractions containing the pure product were pooled and then concentrated to give a yellow solid. This solid was taken up in DCM (1 mL):TFA (0.5 mL); the solution was cooled to 0 °C; and to the solution was added triflic acid (0.057 mL, 0.637 mmol). The mixture was stirred for 1 h and then concentrated *in vacuo.* The residue was taken up in ethyl acetate; washed with 1 N NaOH; washed with 0.5M citric acid; dried over Na₂SO₄; filtered; and then was concentrated *in vacuo.* The residue was subjected to silica gel chromatography (24 g RediSep Gold column) using 0-60 % ethyl acetate in hexanes over 20 CV, then at 60 % ethyl acetate for 10 CV. Fractions containing the pure product were pooled and then concentrated *in vacuo* to give N-(1-((6P)-3-(4-chloro-1-methyl-3-(methylsulfonamido)-1H-indazol-7-yl)-4-oxo-7-(3,3,3-trifluoropropoxy)-3,4-dihydropyrido[2,3-d]pyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide (0.078 g, 0.081 mmol, 38.0 % yield) as a brown solid. ¹H NMR (500 MHz, METHANOL-d₄) δ ppm 8.46 - 8.53 (m, 1 H) 7.28 - 7.34 (m, 1 H) 7.19 - 7.24 (m, 1 H) 7.03 - 7.09 (m, 1 H) 6.53 - 6.81 (m, 4 H) 4.80 (dd, J=5.96, 2.98 Hz, 3 H) 4.49 - 4.62 (m, 2 H) 3.58 - 3.62 (m, 3 H) 3.40 - 3.49 (m, 1 H) 3.22 - 3.24 (m, 3 H) 3.06 - 3.14 (m, 1 H) 2.80 - 2.89 (m, 2 H) 2.37 - 2.44 (m, 2 H) 1.32 - 1.37 (m, 1 H) 0.96 - 1.01 (m, 1 H). LCMS Analysis Method: Column = Acquity UPLC BEH C18, 2.1 x 100 mm, 1.7 µm particles; Injection Volume = 5.00 µL; Flowrate = 0.80 mL/min; Solvent A = 95:5 Water:MeCN w/ 0.1% v/v formic acid; Solvent B = 5:95 Water:MeCN w/ 0.1% v/v formic acid; Elution profile = Start %B: 0, End %B: 100, Gradient Time: 3.5 min. then hold at 100% B for 1 min.; Detection wavelength 1 = 220 nm, wavelength 2 = 254 nm. LCMS retention time = 3.097 min; m/z = 918.05 [M+1]⁺.

### Naming of Example 4:

The compound of Example 4 as prepared above is a homochiral material that contains axial chirality. Axial chirality can be described using P/M nomenclature as detailed in the IUPAC Gold Book (doi:10.1351/goldbook.A00547). However, at this time only a limited number of software tools are available which are capable of generating chemical names containing P/M nomenclature, and even fewer options are available to convert chemical names using this nomenclature to structural representations of the molecules. Therefore, for clarity and convenience several names for Example 4 are provided below:
The name of Example 4 as generated by ChemDraw Professional 16 (absent P/M nomenclature) is:
   N-((S)-1-(3-(4-chloro-1-methyl-3-(methylsulfonamido)-1H-indazol-7-yl)-4-oxo-7-(3,3,3-trifluoropropoxy)-3,4-dihydropyrido[2,3-d]pyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide
The chemical name of Example 4 as generated by JChem for Excel (including P/M nomenclature) is:
   N-[(1S)-1-[(3P,3P)-3-(4-chloro-3-methanesulfonamido-1-methyl-1H-indazol-7-yl)-4-oxo-7-(3,3,3-trifluoropropoxy)-3H,4H-pyrido[2,3-d]pyrimidin-2-yl]-2-(3,5-difluorophenyl)ethyl]-2-[(2S,4R)-9-(difluoromethyl)-5,5-difluoro-7,8-diazatricyclo[4.3.0.0²,⁴]nona-1(6),8-dien-7-yl]acetamide
The chemical name of Example 4 generated by ChemDraw Professional 16 with manually added P/M nomenclature is:
   N-((S)-1-((3P)-3-(4-chloro-1-methyl-3-(methylsulfonamido)-1H-indazol-7-yl)-4-oxo-7-(3,3,3-trifluoropropoxy)-3,4-dihydropyrido[2,3-d]pyrimidin-2-yl)-2-(3,5-difluorophenyl)ethyl)-2-((3bS,4aR)-3-(difluoromethyl)-5,5-difluoro-3b,4,4a,5-tetrahydro-1H-cyclopropa[3,4]cyclopenta[1,2-c]pyrazol-1-yl)acetamide

### Biological Methods:

HIV cell culture assay - MT-2 cells, 293T cells and the proviral DNA clone of NL₄₋₃ virus were obtained from the NIH AIDS Research and Reference Reagent Program. MT-2 cells were propagated in RPMI 1640 media supplemented with 10% heat inactivated fetal bovine serum (FBS), 100 mg/ml penicillin G and up to 100 units/mL streptomycin. The 293T cells were propagated in DMEM media supplemented with 10% heat inactivated FBS, 100 mg/mL penicillin G and 100 mg/mL streptomycin. A recombinant NL₄₋₃ proviral clone, in which a section of the nef gene was replaced with the Renilla luciferase gene, was used to make the reference virus used in these studies. The recombinant virus was prepared through transfection of the recombinant NL₄₋₃ proviral clone into 293T cells using Transit-293 Transfection Reagent from Mirus Bio LLC (Madison, WI). Supernatent was harvested after 2-3 days and the amount of virus present was titered in MT-2 cells using luciferase enzyme activity as a marker by measuring luciferase enzyme activity. Luciferase was quantitated using the EnduRen Live Cell Substrate from Promega (Madison, WI). Antiviral activities of compounds toward the recombinant virus were quantified by measuring luciferase activity in MT-2 cells infected for 4-5 days with the recombinant virus in the presence of serial dilutions of the compound.

The 50% effective concentration (EC₅₀) was calculated by using the exponential form of the median effect equation where (Fa) = 1/[1+ (ED₅₀/drug conc.)m] (Johnson VA, Byington RT. Infectivity Assay. In Techniques in HIV Research. ed. Aldovini A, Walker BD. 71-76. New York: Stockton Press.1990). The 50% inhibitory concentration (EC₅₀) was calculated by using the exponential form of the median effect equation where percent inhibition = 1/[1 + (EC₅₀/drug concentration)*m*], where *m is* a parameter that reflects the slope of the concentration-response curve.

Compound cytotoxicity and the corresponding CC₅₀ values were determined using the same protocol as described in the antiviral assay except that uninfected cells were used. Cytotoxicity was assessed on day 4 in uninfected MT2 cells by using a XTT (2,3-bis[2-Methoxy-4-nitro-5-sulfophenyl]-2H-tetrazolium-5-carboxyanilide inner salt)-based colorimetric assay (Sigma-Aldrich, St Louis, Mo).

| **Example** | **EC₅₀ nM** | **CC₅₀ µM** |
|---|---|---|
| Example 1 | 0.093 | > 20 |
| Example 2 | 0.043 | > 20 |
| Example 3 | 0.019 | > 20 |
| Example 4 | 0.025 | > 0.5 |

### Procedure for measuring pharmacokinetic parameters in a subcutaneous in vivo experiment (Formulation A):

To a 20 mL vial charged with Example 1 (320 mg) was added PEG 300 (0.906 mL). The mixture was sonicated to afford a clear solution. To the solution was added water (0.160 mL) to afford a slightly hazy solution. The mixture was sonicated for 5 minutes to afford a clear solution. The resulting solution is "Formulation A" and the concentration of the solution is 21.3 w/w% Example 1, 68.1 w/w% PEG 300, and 10.6 w/w% water.

"Formulation A" was administered to Wistar Han Rats as a subcutaneous injection at a dose of 0.167 mL/kg. Blood samples were collected at 0.5, 1, 3, 5, 7, 24, 48, 72 hours post-dose and on Day 6, 8, 12, 15, 19, 22, 26, 29, 33, 36, 40, 43, 47, 50, 54, 57, 61. Blood samples were collected into K₂EDTA tubes and centrifuged at 1500 to 2000 x g to obtain plasma. Plasma samples were stored at -80°C until analysis by LC-MS/MS. All *in vitro* samples were injected on a MDS Sciex API 4000 triple-quadrupole LC-MS/MS system. The analytical column used was a Phenomenex Kinetex^{®} 2.6 µm PS (C18, 2.1mm x 50 mm, 2.6 µm) maintained at room temperature. Mobile Phase A consisted of 0.1% (v/v) formic acid in MilliQ-purified water. Mobile Phase B consisted of 0.1% (v/v) formic acid in acetonitrile. The flow rate was 0.70 mL/min. The gradient was as follows: Mobile B was held for 0.5 minutes at 35% and then linearly increased from 35% to 98% over 1.5 min, maintained at 98% for 0.5 min, and maintained at 35% for 0.5 min. Results of the PK experiment are described in Table 1 and Figure 1.

**Table 1. Plasma concentration vs. time data for a study evaluating "Formulation A" administered to rats subcutaneously at a dose of 0.167 mL/kg (n = 3).**

| Days | Hours | Rat 1 (ng/mL) | Rat 2 (ng/mL) | Rat 3 (ng/mL) | Avg. Conc. (ng/mL) | Std. Dev. Conc. (ng/mL) |
|---|---|---|---|---|---|---|
| 0.02 | 0.5 | <LOD | <LOD | <LOD | | |
| 0.04 | 1 | <LOD | 1.5 | <LOD | 1.5 | |
| 0.13 | 3 | 5.2 | 4.6 | 7.9 | 5.9 | 1.8 |
| 0.21 | 5 | 9.0 | 9.0 | 18.3 | 12.1 | 5.4 |
| 0.29 | 7 | 14.1 | 12.2 | 20.4 | 15.6 | 4.3 |
| 1 | 24 | 32.7 | 29.0 | 43.1 | 34.9 | 7.3 |
| 2 | 48 | 42.7 | 32.8 | 57.8 | 44.4 | 12.6 |
| 3 | 72 | 36.5 | 29.8 | 42.1 | 36.1 | 6.2 |
| 5 | 120 | 29.1 | 40.9 | 35.1 | 35.0 | 5.9 |
| 7 | 168 | 36.6 | 49.8 | 29.0 | 38.5 | 10.5 |
| 10 | 240 | 86.3 | 75.9 | 51.0 | 71.1 | 18.1 |
| 14 | 336 | 111.0 | 82.7 | 58.7 | 84.1 | 26.2 |

| | | | | | | |
|---|---|---|---|---|---|---|
| LOD = Limit of detection | | | | | | |

The disclosure is not limited to the foregoing illustrative examples and the examples should be considered in all respects as illustrative and not restrictive, reference being made to the appended claims, rather than to the foregoing examples.

## Claims

1. A pharmaceutical composition comprising the compound of Formula Ia, Formula Ib, Formula Ic, or Formula Id: 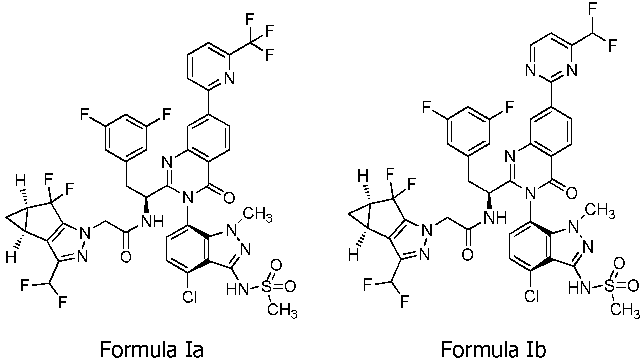 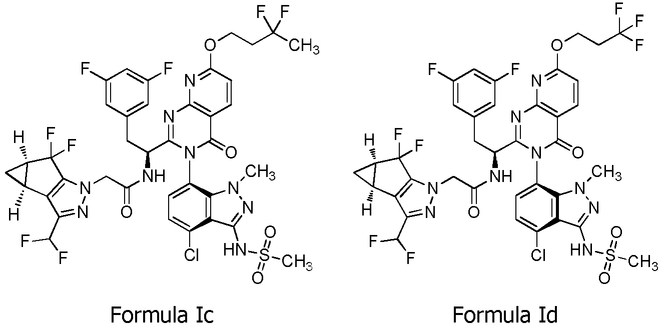 or a pharmaceutically acceptable salt thereof and a solvent or diluent selected from the group consisting of water, an alcohol, polyethylene glycol (PEG), N-methyl-2-pyrrolidone (NMP), ethyl lactate, propylene glycol, glycofurol, and dimethylsulfoxide (DMSO), wherein the compound of Formula Ia, Formula Ib, Formula Ic, Formula Id, or pharmaceutically acceptable salt thereof, is present in the composition at a concentration of 50 to 500 mg/mL, based on weight of a free compound of Formula Ia, Formula Ib, Formula Ic, Formula Id.

2. A pharmaceutical composition according to Claim 1 further comprising an excipient selected from the group consisting of polysorbate 20, polysorbate 80, poloxamer 188, poloxamer 207, poloxamer 338, sodium chloride, and sodium hydroxide.

3. A pharmaceutical composition according to Claim 1 or Claim 2 which is a solution.

4. A pharmaceutical composition according to Claim 1 or Claim 2 which is a suspension.

5. A pharmaceutical composition according to any of Claims 1-4 comprising polyethylene glycol (PEG).

6. A pharmaceutical composition according to Claim 5 wherein the average molecular weight of PEG is about 300 (PEG 300).

7. A pharmaceutical composition according to any of Claims 1-6 comprising ethanol; and/or comprising poloxamer 188.

8. A pharmaceutical composition according to any of Claims 1-7 comprising sodium hydroxide.

9. A pharmaceutical composition according to any of Claims 2-8 wherein at least 90 % by weight of the solvents or diluents are water and PEG 300.

10. A pharmaceutical composition according to any of Claims 2-9 wherein at least 90 % by weight of the excipient is poloxamer 188.

11. A pharmaceutical composition according to Claims 9 wherein the water is present in the composition at 8 to 20 % by weight and the PEG 300 is present in the composition at 60-85 % by weight.

12. A pharmaceutical composition according to Claim 11 wherein water is present in the composition at 8 to 12 % by weight and the PEG 300 is present in the composition at 63-70 % by weight.

13. A pharmaceutical composition according to any of Claims 1-4, wherein the composition comprises water.

14. A pharmaceutical composition according to Claim 13 wherein the compound of Formula Ia, Formula Ib, Formula Ic, or Formula Id, or a pharmaceutically acceptable salt thereof, is present in the composition at a concentration of 225 to 275 mg/mL, based on weight of the free compound of Formula Ia, Formula Ib, Formula Ic, or Formula Id.

15. A pharmaceutical composition according to Claim 13 wherein the compound of Formula Ia, Formula Ib, Formula Ic, or Formula Id, or a pharmaceutically acceptable salt thereof, is present in the composition at a concentration of 275 to 350 mg/mL, based on weight of the free compound of Formula Ia, Formula Ib, Formula Ic, or Formula Id; or
wherein the compound of Formula Ia, Formula Ib, Formula Ic, or Formula Id, or a pharmaceutically acceptable salt thereof, is present in the composition at a concentration of 350 to 425 mg/mL, based on weight of the free compound of Formula Ia, Formula Ib, Formula Ic, or Formula Id.

16. The pharmaceutical composition according to claim 1, wherein the composition comprises a compound of Formula Id or pharmaceutically acceptable salt thereof.

17. The pharmaceutical composition according to claim 1, wherein the solvent or diluent comprises polyethylene glycol having an average molecular weight of about 400 (PEG 400).

18. The pharmaceutical composition according to claim 1, further comprising an acetate buffer.

19. The pharmaceutical composition according to claim 1, further comprising docusate sodium.

## Patentansprüche

1. Pharmazeutische Zusammensetzung, umfassend die Verbindung der Formel Ia, Formel Ib, Formel Ic oder Formel Id: 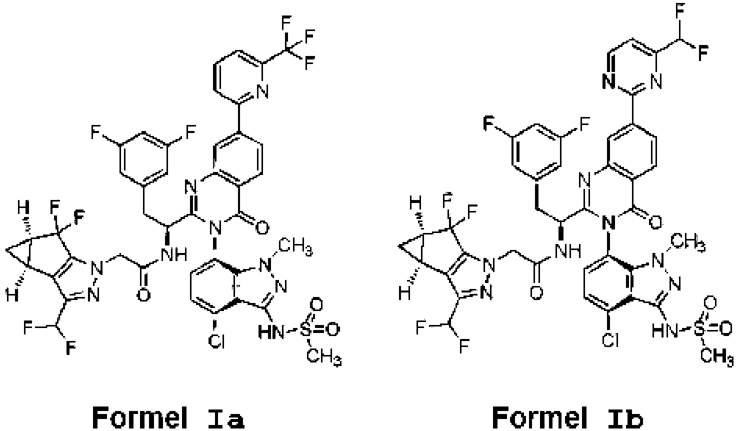 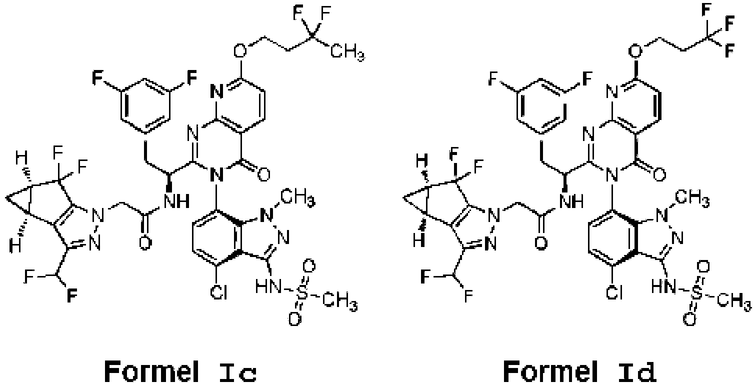 oder ein pharmazeutisch annehmbares Salz davon und ein Lösungsmittel oder Verdünnungsmittel, ausgewählt aus der Gruppe, bestehend aus Wasser, einem Alkohol, Polyethylenglykol (PEG), N-Methyl-2-pyrrolidon (NMP), Ethyllactat, Propylenglykol, Glycofurol und Dimethylsulfoxid (DMSO), wobei die Verbindung der Formel Ia, Formel Ib, Formel Ic, Formel Id oder das pharmazeutisch annehmbare Salz davon in der Zusammensetzung in einer Konzentration von 50 bis 500 mg/mL, bezogen auf das Gewicht einer freien Verbindung der Formel Ia, Formel Ib, Formel Ic, Formel Id, vorhanden ist.

2. Pharmazeutische Zusammensetzung gemäß Anspruch 1, ferner umfassend einen Hilfsstoff, ausgewählt aus der Gruppe, bestehend aus Polysorbat 20, Polysorbat 80, Poloxamer 188, Poloxamer 207, Poloxamer 338, Natriumchlorid und Natriumhydroxid.

3. Pharmazeutische Zusammensetzung gemäß Anspruch 1 oder Anspruch 2, die eine Lösung ist.

4. Pharmazeutische Zusammensetzung gemäß Anspruch 1 oder 2, die eine Suspension ist.

5. Pharmazeutische Zusammensetzung gemäß einem der Ansprüche 1-4, umfassend Polyethylenglykol (PEG).

6. Pharmazeutische Zusammensetzung gemäß Anspruch 5, wobei das durchschnittliche Molekulargewicht von PEG etwa 300 (PEG 300) beträgt.

7. Pharmazeutische Zusammensetzung gemäß einem der Ansprüche 1-6, umfassend Ethanol und/oder umfassend Poloxamer 188.

8. Pharmazeutische Zusammensetzung gemäß einem der Ansprüche 1-7, umfassend Natriumhydroxid.

9. Pharmazeutische Zusammensetzung gemäß einem der Ansprüche 2-8, wobei mindestens 90 Gew.-% der Lösungsmittel oder Verdünnungsmittel Wasser und PEG 300 sind.

10. Pharmazeutische Zusammensetzung gemäß einem der Ansprüche 2-9, wobei mindestens 90 Gew.-% des Hilfsstoffs Poloxamer 188 ist.

11. Pharmazeutische Zusammensetzung gemäß Anspruch 9, wobei das Wasser in der Zusammensetzung zu 8 bis 20 Gew.-% vorhanden ist, und das PEG 300 in der Zusammensetzung zu 60-85 Gew.-% vorhanden ist.

12. Pharmazeutische Zusammensetzung gemäß Anspruch 11, wobei Wasser in der Zusammensetzung zu 8 bis 12 Gew.-% vorhanden ist, und das PEG 300 in der Zusammensetzung zu 63-70 Gew.-% vorhanden ist.

13. Pharmazeutische Zusammensetzung gemäß einem der Ansprüche 1-4, wobei die Zusammensetzung Wasser umfasst.

14. Pharmazeutische Zusammensetzung gemäß Anspruch 13, wobei die Verbindung der Formel Ia, Formel Ib, Formel Ic, Formel Id oder ein pharmazeutisch annehmbares Salz davon in der Zusammensetzung in einer Konzentration von 225 bis 275 mg/mL, bezogen auf das Gewicht der freien Verbindung der Formel Ia, Formel Ib, Formel Ic oder Formel Id, vorhanden ist.

15. Pharmazeutische Zusammensetzung gemäß Anspruch 13, wobei die Verbindung der Formel Ia, Formel Ib, Formel Ic, Formel Id oder ein pharmazeutisch annehmbares Salz davon in der Zusammensetzung in einer Konzentration von 275 bis 350 mg/mL, bezogen auf das Gewicht der freien Verbindung der Formel Ia, Formel Ib, Formel Ic oder Formel Id, vorhanden ist oder
wobei die Verbindung der Formel Ia, Formel Ib, Formel Ic, Formel Id oder ein pharmazeutisch annehmbares Salz davon in der Zusammensetzung in einer Konzentration von 350 bis 425 mg/mL, bezogen auf das Gewicht der freien Verbindung der Formel Ia, Formel Ib, Formel Ic oder Formel Id, vorhanden ist.

16. Pharmazeutische Zusammensetzung gemäß Anspruch 1, wobei die Zusammensetzung eine Verbindung der Formel Id oder ein pharmazeutisch annehmbares Salz davon umfasst.

17. Pharmazeutische Zusammensetzung gemäß Anspruch 1, wobei das Lösungsmittel oder Verdünnungsmittel Polyethylenglykol mit einem durchschnittlichen Molekulargewicht von etwa 400 (PEG 400) umfasst.

18. Pharmazeutische Zusammensetzung gemäß Anspruch 1, ferner umfassend einen Acetatpuffer.

19. Pharmazeutische Zusammensetzung gemäß Anspruch 1, ferner umfassend Docusat-Natrium.

## Revendications

1. Composition pharmaceutique comprenant le composé de formule Ia, de formule Ib, de formule Ic, ou de formule Id : 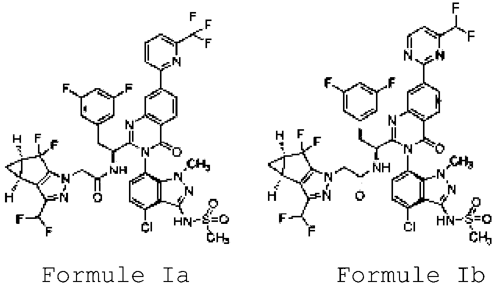 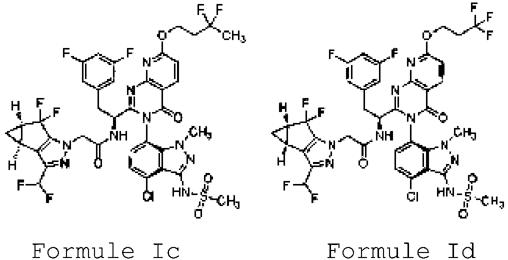 ou un sel pharmaceutiquement acceptable de ce dernier et un solvant ou diluant choisi dans le groupe constitué d'eau, d'un alcool, de polyéthylèneglycol (PEG), de N-méthyl-2-pyrrolidone (NMP), de lactate d'éthyle, de propylèneglycol, de glycofurol et de diméthylsulfoxyde (DMSO), dans laquelle le composé de formule Ia, de formule Ib, de formule Ic, de formule Id, ou un sel pharmaceutiquement acceptable de ce dernier, est présent dans la composition à une concentration de 50 à 500 mg/ml, sur la base d'un poids d'un composé libre de formule Ia, de formule Ib, de formule Ic, de formule Id.

2. Composition pharmaceutique selon la revendication 1, comprenant en outre un excipient choisi dans le groupe constitué de polysorbate 20, de polysorbate 80, de poloxamère 188, de poloxamère 207, de poloxamère 338, de chlorure de sodium et d'hydroxyde de sodium.

3. Composition pharmaceutique selon la revendication 1 ou la revendication 2 qui est une solution.

4. Composition pharmaceutique selon la revendication 1 ou la revendication 2 qui est une suspension.

5. Composition pharmaceutique selon l'une quelconque des revendications 1 à 4, comprenant du polyéthylèneglycol (PEG) .

6. Composition pharmaceutique selon la revendication 5, dans laquelle le poids moléculaire moyen de PEG est d'environ 300 (PEG 300).

7. Composition pharmaceutique selon l'une quelconque des revendications 1 à 6, comprenant de l'éthanol ; et/ou comprenant du poloxamère 188.

8. Composition pharmaceutique selon l'une quelconque des revendications 1 à 7, comprenant de l'hydroxyde de sodium.

9. Composition pharmaceutique selon l'une quelconque des revendications 2 à 8, dans laquelle au moins 90 % en poids des solvants ou diluants sont de l'eau et du PEG 300.

10. Composition pharmaceutique selon l'une quelconque des revendications 2 à 9, dans laquelle au moins 90 % en poids de l'excipient est du poloxamère 188.

11. Composition pharmaceutique selon la revendication 9, dans laquelle l'eau est présente dans la composition à 8 à 20 % en poids et le PEG 300 est présent dans la composition à 60 à 85 % en poids.

12. Composition pharmaceutique selon la revendication 11, dans laquelle l'eau est présente dans la composition à 8 à 12 % en poids et le PEG 300 est présent dans la composition à 63 à 70 % en poids.

13. Composition pharmaceutique selon l'une quelconque des revendications 1 à 4, dans laquelle la composition comprend de l'eau.

14. Composition pharmaceutique selon la revendication 13, dans laquelle le composé de formule Ia, de formule Ib, de formule Ic, ou de formule Id, ou un sel pharmaceutiquement acceptable de ce dernier, est présent dans la composition à une concentration de 225 à 275 mg/ml, sur la base d'un poids du composé libre de formule Ia, de formule Ib, de formule Ic, ou de formule Id.

15. Composition pharmaceutique selon la revendication 13, dans laquelle le composé de formule Ia, de formule Ib, de formule Ic, ou de formule Id, ou un sel pharmaceutiquement acceptable de ce dernier, est présent dans la composition à une concentration de 275 à 350 mg/ml, sur la base d'un poids du composé libre de formule Ia, de formule Ib, de formule Ic, ou de formule Id ; ou
dans laquelle le composé de formule Ia, de formule Ib, de formule Ic, ou de formule Id, ou un sel pharmaceutiquement acceptable de ce dernier, est présent dans la composition à une concentration de 350 à 425 mg/ml, sur la base d'un poids du composé libre de formule Ia, de formule Ib, de formule Ic, ou de formule Id.

16. Composition pharmaceutique selon la revendication 1, dans laquelle la composition comprend un composé de formule Id ou un sel pharmaceutiquement acceptable de ce dernier.

17. Composition pharmaceutique selon la revendication 1, dans laquelle le solvant ou diluant comprend du polyéthylèneglycol ayant un poids moléculaire moyen d'environ 400 (PEG 400).

18. Composition pharmaceutique selon la revendication 1, comprenant en outre un tampon acétate.

19. Composition pharmaceutique selon la revendication 1, comprenant en outre du docusate de sodium.
